(19) **Europäisches Patentamt European Patent Office Office européen des brevets**

(11) **EP 4 324 817 B1**

(12) **EUROPEAN PATENT SPECIFICATION**

(45) Date of publication and mention of the grant of the patent:
**28.01.2026 Bulletin 2026/05**

(21) Application number: **22899121.2**

(22) Date of filing: **28.11.2022**

(51) International Patent Classification (IPC):
**C07C 59/01** (2006.01)     **C07C 51/48** (2006.01)
**C07C 51/43** (2006.01)     **C12P 7/52** (2006.01)
**C12N 9/88** (2006.01)     **C07C 51/02** (2006.01)

(52) Cooperative Patent Classification (CPC):
(C-Sets available)
**C07C 51/43; C07C 51/02; C07C 51/412;**
**C07C 59/01; C12N 9/0008; C12N 9/88; C12P 7/52;**
C12R 2001/19                                      (Cont.)

(86) International application number:
**PCT/KR2022/018943**

(87) International publication number:
**WO 2023/096439 (01.06.2023 Gazette 2023/22)**

(54) **PROCESS OF RECOVERING 3-HYDROXYPROPIONIC ACID AND SLURRY COMPOSITION COMPRISING 3-HYDROXYPROPIONIC ACID**

VERFAHREN ZUR RÜCKGEWINNUNG VON 3-HYDROXYPROPIONSÄURE UND SCHLAMMZUSAMMENSETZUNG MIT 3-HYDROXYPROPIONSÄURE

PROCÉDÉ DE RÉCUPÉRATION D'ACIDE 3-HYDROXYPROPIONIQUE ET COMPOSITION DE SUSPENSION COMPRENANT DE L'ACIDE 3-HYDROXYPROPIONIQUE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **29.11.2021   KR 20210166976**
**29.11.2021   KR 20210166978**
**29.11.2021   KR 20210167303**
**29.11.2021   KR 20210167409**
**24.11.2022   KR 20220159392**
**24.11.2022   KR 20220159394**
**24.11.2022   KR 20220159395**

(43) Date of publication of application:
**21.02.2024   Bulletin 2024/08**

(73) Proprietor: **LG Chem, Ltd.**
**Seoul 07336 (KR)**

(72) Inventors:
• **KIM, Jeong Eun**
  **Daejeon 34122 (KR)**
• **BANG, Yongju**
  **Daejeon 34122 (KR)**

• **CHOE, Jae Hoon**
  **Daejeon 34122 (KR)**
• **KANG, Donggyun**
  **Daejeon 34122 (KR)**
• **CHO, Ara**
  **Daejeon 34122 (KR)**
• **JUNG, Woochul**
  **Daejeon 34122 (KR)**
• **HAN, Sang Won**
  **Daejeon 34122 (KR)**
• **KIM, Taeho**
  **Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

(56) References cited:
**WO-A1-2015/166098     US-B2- 8 883 464**

EP 4 324 817 B1

- URBANUS J ET AL: "Intensified crystallization in complex media: Heuristics for crystallization of platform chemicals", CHEMICAL ENGINEERING SCIENCE, OXFORD, GB, vol. 77, 13 February 2012 (2012-02-13), pages 18 - 25, XP028500286, ISSN: 0009-2509, [retrieved on 20120224], DOI: 10.1016/J.CES.2012.02.019
- J. URBANUS; C.P.M. ROELANDS; D. VERDOES; J.H. TER HORST: "Intensified crystallization in complex media: Heuristics for crystallization of platform chemicals", CHEMICAL ENGINEERING SCIENCE, vol. 77, 13 February 2012 (2012-02-13), GB
  , pages 18 - 25, XP028500286, ISSN: 0009-2509, DOI: 10.1016/j.ces.2012.02.019
- MCDONALD MATTHEW A, HOSSEIN SALAMI, PATRICK R. HARRIS, COLTON E. LAGERMAN, XIAOCHUAN YANG, ANDREAS S. BOMMARIUS, MARTHA A. GROVER,: "Reactive crystallization: a review", REACTION CHEMISTRY & ENGINEERING, vol. 6, no. 3, 1 March 2021 (2021-03-01), pages 364 - 400, XP055951360, ISSN: 2058-9883, DOI: 10.1039/D0RE00272K
- SUMITRA RAMACHANDRAN , PIERRE FONTANILLE , ASHOK PANDEY , CHRISTIAN LARROCHE: "Gluconic acid: Properties, applications and microbial production", FOOD TECHNOLOGY AND BIOTECHNOLOGY, vol. 44, no. 2, 1 April 2006 (2006-04-01), Croatia
  , pages 185 - 195, XP002670029, ISSN: 1330-9862

(52) Cooperative Patent Classification (CPC): (Cont.)

C-Sets
C07C 51/02, C07C 59/01;
C07C 51/412, C07C 59/01;
C07C 51/43, C07C 59/01

**Description**

## FIELD OF THE INVENTION

The present invention relates to a process of recovering 3-hydroxypropionic acid and a slurry composition containing 3-hydroxypropionic acid.

## BACKGROUND OF THE INVENTION

3-Hydroxypropionic acid (3HP) is a platform compound that can be converted to various chemicals such as acrylic acid, methyl acrylate, and acrylamide. Since being identified as one of the Top 12 value-added bio-chemicals by the US Department of Energy (DOE) in 2004, it has been actively researched in academic and industrial world.

The production of 3-hydroxypropionic acid is mainly carried out by two methods, chemical and biological, but in the case of chemical method, it has been pointed out that the initial material is expensive and that it is non-environmental because toxic substances are generated during the production process, and thus, environmentally friendly bioprocesses have been attracting attention.

When organic acids are produced by microbial fermentation, other by-products are produced in addition to organic acids such as 3-hydroxypropionic acid in the process of fermenting microorganisms, and thus, a process of extracting and separating organic acids from the fermentation liquor is needed. As methods for extracting and separating organic acids from microbial fermentation liquid, an electrodialysis method, a reverse osmosis membrane method, an organic acid-containing solution-organic solvent reaction extraction method, and the like are used. In particular, the back extraction method using sodium hydroxide (NaOH) is widely used because of its high yield. However, in the case of these methods, since the product is in the form of an organic acid salt, there is a disadvantage that a process for converting it to an organic acid is further needed, and the purity is low.

3-Hydroxypropionic acid exhibits high hydrophilicity and has high solubility and reactivity with water, unlike other organic acids produced through fermentation processes. This makes it difficult to apply conventional organic acid separation and purification processes such as precipitation and extraction.

The reactive extraction method is a method of extracting an organic acid using an active diluent such as an amine or an alcohol that is highly reactive with the organic acid, which method allows selective extraction of organic acid, and has a relatively high extraction efficiency. Accordingly, an attempt has been made to apply the reaction extraction method even when separating and purifying 3HP. As an example, a method using trioctylamine (TOA) as the amine has been proposed, but there are problems that the extraction efficiency of 3HP is low and a large amount of organic solvent is required. Further, when using tridecylamine as an amine, the extraction efficiency of 3HP is higher than that of TOA, but there is a problem that an emulsion phenomenon occurs in which an organic phase and an aqueous phase are not separated during extraction.

Therefore, there is a need to develop a process of recovering 3-hydroxypropionic acid in a high purity and a high yield from a raw material solution containing 3-hydroxypropionic acid, such as a microbial fermentation liquid.

Chemical Engineering Science, vol. 77, pages 18-25, discloses the separation of 3-hydroxypropionic acid from a fermentation mixture and its purification by using a variety of techniques including co-crystallization and electrochemically-induced crystallization.

## SUMMARY OF THE INVENTION

## TECHNICAL PROBLEM

It is an object of the present invention to provide a process of recovering 3-hydroxypropionic acid in which the washing liquid, which has been used for washing the precipitate formed by reacting the 3-hydroxypropionate crystal with the acid, is reused during the process to thereby recover 3-hydroxypropionic acid in a high purity and a high yield, and a slurry composition comprising the precipitate formed in the above process and 3-hydroxypropionic acid.

## TECHNICAL SOLUTION

Provided herein is a process of recovering 3-hydroxypropionic acid, comprising: forming a 3-hydroxypropionate crystal in a concentrate containing 3-hydroxypropionic acid in the presence of an alkali metal salt or an alkali earth metal salt; preparing a solution containing 3-hydroxypropionate crystal separated from the concentrate; stirring an acid and the solution containing 3-hydroxypropionate crystal to form a precipitate; and subjecting the precipitate to a first washing.

Also provided herein is a slurry composition comprising a precipitate represented by the following structural formula 4 and 3-hydroxypropionic acid.

[Structural Formula 4]          $Cation(Anion) \cdot pH_2O$

wherein,

the Cation is $Na^+$, $Mg^{2+}$ or $Ca^{2+}$,
the Anion is $SO_4^{2-}$, $PO_4^{3-}$ or $CO_3^{2-}$, and
the p is the number of water molecules in a hydrate, which is an integer of 1 or more.

Further embodiments are disclosed in the dependent claims.

Now, a process of recovering 3-hydroxypropionic acid and a slurry composition comprising 3-hydroxypropionic acid according to embodiments of the present invention will be described in more detail.

It should be understood that, unless steps included in a preparation method described herein are specified as being sequential or consecutive or otherwise stated, one step and another step included in the preparation method should not be construed as being limited to an order described herein. Therefore, it should be understood that an order of steps included in a preparation method can be changed within the range of understanding of those skilled in the art,

In the present invention, the alkali metal includes both alkali metal and alkali earth metal.

According to one embodiment of the present invention, there is provided a process of recovering 3-hydroxypropionic acid, comprising: forming a 3-hydroxypropionate crystal in a concentrate containing 3-hydroxypropionic acid in the presence of an alkali metal salt or an alkali earth metal salt; preparing a solution containing 3-hydroxypropionate crystal separated from the concentrate; stirring an acid and the solution containing 3-hydroxypropionate crystal to form a precipitate; and subjecting the precipitate to a first washing.

Also, the process of recovering 3-hydroxypropionic acid according to one embodiment of the invention may further comprise reusing a first washing liquid used for the first washing of the precipitate.

The present inventors have found through experiments that when 3-hydroxypropionic acid is concentrated in the presence of an alkali metal salt or an alkali earth metal salt to form 3-hydroxypropionate crystal, the solution containing 3-hydroxypropionate crystal and the acid are stirred, the resulting precipitate is separated and washed, and the used washing liquid is reused during the process, the loss of 3-hydroxypropionic acid in the process can be prevented, and the 3-hydroxypropionic acid can be recovered at a high concentration and a high recovery rate, and completed the present invention.

When the solution containing 3-hydroxypropionate crystal reacts with an acid, a precipitate may be formed. For example, when 3-hydroxypropionate is $Ca(3HP)_2$ and sulfuric acid is used as an acid, a reaction as in the following Reaction Scheme 1 can proceed to produce a slurry composition containing a precipitate ($CaSO_4 \cdot 2H_2O$) and 3-hydroxypropionic acid. Additionally, the precipitate can be filtered from such a slurry composition to thereby recover 3-hydroxypropionic acid (3HP) contained in the filtrate.

[Reaction Scheme 1]          $Ca(3HP)_2 + H_2SO_4 + 2H_2O \rightarrow CaSO_4 \cdot 2H_2O + 2(3HP)$

Meanwhile, precipitates such as $CaSO_4 \cdot 2H_2O$ have the property of well absorbing water, and thus, there is a problem that a large amount of 3-hydroxypropionic acid remains in the precipitate together with water, and the 3-hydroxypropionic acid is lost. In order to reduce the loss of 3-hydroxypropionic acid, the precipitate can be washed with a large amount of water, but in this case, the problem is that the concentration of 3-hydroxypropionic acid in the final product solution is very low.

However, in the process of recovering 3-hydroxypropionic acid according to one embodiment of the invention, the washing liquid used for washing the precipitate is reused during the process of recovering 3-hydroxypropionic acid, which can prevent loss of 3-hydroxypropionic acid, and can also prevent the concentration of 3-hydroxypropionic acid from decreasing in the final product solution, so that 3-hydroxypropionic acid can be recovered at high concentration and high recovery rate.

The reuse of the washing liquid means that the washing liquid used for washing the precipitate is reused during the recovery process of the 3-hydroxypropionic acid. For example, in the process of recovering 3-hydroxypropionic acid, the washing liquid can be used again as a liquid material in the step where the liquid material is required, and the reuse may include not only reusing the washing liquid before the precipitate forming step, but also reusing the washing liquid after the precipitate forming step.

For example, when the washing liquid is used again before the precipitate forming step, the washing liquid can be reused as a solvent in the step of preparing a solution containing 3-hydroxypropionate crystal, and the washing liquid can be reused in the fermentation liquid in the step of fermenting a bacterium strain having 3-hydroxypropionic acid-producing ability to produce a 3-hydroxypropionic acid fermentation liquor. In addition, when the washing liquid is used again after the precipitate forming step, the washing liquid is mixed with the filtrate in which the precipitate has been filtered to prepare a mixed liquid, and the 3-hydroxypropionic acid (3HP) can be recovered from the mixed liquid.

**In** the process of recovering 3-hydroxypropionic acid according to one embodiment of the invention, after forming the 3-hydroxypropionate crystal, the 3-hydroxypropionate crystal is separated from the concentrate, and a solution containing 3-hydroxypropionate crystal separated from the concentrate can be prepared.

As mentioned above, as the 3-hydroxypropionate crystal are produced in the fermentation process, the concentrate in which the fermentation liquid has been concentrated may contain a large amount of impurities such as bacterium strains, carbon sources, and alkali metal salts or alkali earth metal salts in addition to the 3-hydroxypropionate crystal. However, in the process of recovering 3-hydroxypropionic acid according to one embodiment of the invention, the impurities can be removed by solid-liquid separation and recovery of 3-hydroxypropionate crystal from the concentrate, and the impurities may not be contained even in a solution prepared by dissolving the separated 3-hydroxypropionic acid crystals in a solvent such as distilled water.

For example, the 3-hydroxypropionate crystal can be separated from the concentrate using a filtration flask, a vacuum pump, or the like, and the separated 3-hydroxypropionate crystal can be dissolved in a solvent such as distilled water to produce a solution containing 3-hydroxypropionate crystal.

The solution containing 3-hydroxypropionate crystal may contain 3-hydroxypropionate crystal at a concentration of 100 g/L or more, 150 g/L or more, or 200 g/L or more, and may contain it at a concentration of 800 g/L or less, 750 g/L or less, or 700 g/L or less. If the concentration of the 3-hydroxypropionate crystal contained in the solution containing 3-hydroxypropionate crystal is too low, the concentration of 3-hydroxypropionic acid finally recovered may be lowered, and if the concentration of the 3-hydroxypropionate crystal is too high, the recovery rate 3-hydroxypropionic acid may be reduced.

In the process of recovering 3-hydroxypropionic acid, after preparing the solution containing 3-hydroxypropionate crystal, the acid and the solution containing 3-hydroxypropionate crystal can be stirred to prepare a slurry containing a precipitate and 3-hydroxypropionic acid.

The step of stirring the acid and the solution containing 3-hydroxypropionate crystal is not limited thereto, but may further comprise adding an acid to the solution containing 3-hydroxypropionate crystal at a temperature of 30°C or more and 90°C or less, before the step of stirring the acid and the solution containing 3-hydroxypropionate crystal to form a precipitate.

Since the acid is added to the 3-hydroxypropionate crystal at the temperature of 30°C or more and 90°C or less, the particle size of the precipitate formed is large, and the moisture content is also significantly low, so that the fluidity of the slurry composition containing the same can be increased. Thereby, the filtration of a precipitate from the slurry composition is facilitated, and 3-hydroxypropionic acid can be recovered at a high recovery rate.

Meanwhile, the temperature when adding an acid to the 3-hydroxypropionate crystal may be 30°C or more, 35°C or more, 40°C or more, 45°C or more, 50°C or more, 55°C or more, and may be 90°C or less, 80°C or less, 75°C or less, 70°C or less, or 65°C or less.

The size and moisture content of the precipitate appears to be affected by the temperature conditions of the acid adding step. When an acid is added to the 3-hydroxypropionate crystal under the above temperature conditions, the precipitate may have large particle size and low moisture content.

Further, before the step of stirring an acid and the solution containing 3-hydroxypropionate crystal to form a precipitate, the process may further comprise charging the acid and the solution containing 3-hydroxypropionate crystal into a reactor, either continuously or in a state divided in 2 times or more.

Since the acid and the solution containing 3-hydroxypropionate crystal are charged into a reactor, either continuously or in a state divided in 2 times or more, the particle size of the precipitate formed is as large as 10.0 $\mu$m or more, and the moisture content may also be remarkably low. Thereby, the filtration of the precipitate from the slurry composition is facilitated, and as the fluidity of the slurry composition is increased, the filtration of the precipitate from the slurry composition is facilitated, and 3-hydroxypropionic acid can be recovered at a high recovery rate. **In** addition, since the particle size of the precipitate is as large as 10.0 $\mu$m or more, not only filtration is remarkably facilitated, but also the dust generated when recycling the separated precipitate to other industrial fields such as cement production is reduced, which can be very advantageous in processing.

As the concentration of the reactants contained in the reactor is kept smaller, the particle size of the precipitate can be formed larger. In order to keep the concentration of the reactants low in the reactor, the acid and the solution containing 3-hydroxypropionate crystal, which are the reactants, may be continuously charged into the reactor, or may be charged in a state divided in 2 times or more.

When continuously charging an acid and/or a 3-hydroxypropionate crystal into a reactor, it can be charged into the reactor at a rate of 0.01 g/min or more, 0.05 g/min or more, or 0.10 g/min or more, and may be charged at a rate of 5.00 g/min or less, 3.00 g/min or less, 1.00 g/min or less, or 0.50 g/min or less. Further, when continuously charging an acid or a 3-hydroxypropionate crystal into a reactor, it may be continuously charged for 10 minutes or more, 20 minutes or more, 30 minutes or more, or 50 minutes or more, and may be continuously charged for 10 hours or less, 5 hours or less, or 2 hours or less. Since the acid and/or the 3-hydroxypropionate crystal are continuously charged into the reactor for the above-mentioned rate and time, a precipitate having a large particle size of 10.0 $\mu$m or more may be formed.

For example, in the process of recovering 3-hydroxypropionic acid, the solution containing 3-hydroxypropionate crystal may be continuously charged into the reactor, and may be continuously charged at a rate of 0.01 g/min or more and 5.00 g/min or less for a time of 10 minutes or more and 10 hours or less.

Further, the acid and/or the 3-hydroxypropionate crystal may be charged into the reactor in a state divided in 2 times or more, 3 times or more, 4 times or more, or 5 times or more, and may be charged in a state divided into 20 times or less, 15 times or less, 13 times or less, or 10 times or less. At this time, the content of the reactants charged at each stage can be controlled to be identical or different, and the same content of reactants may be added to each stage in order to allow the reaction to proceed uniformly.

Further, when the acid and/or the 3-hydroxypropionate crystal are charged in a state divided in 2 times or more, they may be charged at intervals of 1 minute or more, 3 minutes or more, or 5 minutes or more, and may be charged at intervals of 30 minutes or less, 25 minutes or less, 20 minutes or less, or 15 minutes or less. The acid and/or the solution containing 3-hydroxypropionate crystal are divisionally charged into the reactor at the above-mentioned stages and charging intervals, so that a large precipitate having a particle size of 10.0 $\mu$m or more can be formed.

For example, in the process of recovering 3-hydroxypropionic acid, the acid may be charged into the reactor in a state divided in 3 times or more and 20 times or less, actor, and the acid may be divisionally charged into the reactor at intervals of one minute or more and 30 minutes or less.

The temperature inside the reactor may be 20°C or more, 30°C or more, 35°C or more, 40°C or more, 45°C or more, 50°C or more, or 55°C or more, and may be 90°C or less, 80°C or less, 75°C or less, 70°C or less, or 65°C or less. The temperature inside the reactor satisfies the above range, so that the particle size of the precipitate formed may be large and the moisture content may be low.

Meanwhile, the total content of the acid charged into the reactor may be 20 wt.% or more, or 30 wt. % or more, and may be 80 wt. % or less, 70 wt. % or less, 60 wt. % or less, or 50 wt. % or less with respect to 100 wt. % of the total content of the 3-hydroxypropionate crystal. When the content of the acid added is too small, the conversion rate from 3-hydroxypropionate crystal to 3-hydroxypropionic acid is low, and thus, the content of 3-hydroxypropionic acid recovered may become small. When the content of the acid added is too large, the concentration of unreacted cations and anions remaining in the finally obtained 3-hydroxypropionic acid aqueous solution becomes too high, which may adversely affect the progress of subsequent processes such as preparation process of polylactic acid (PLA), preparation process of bio-acrylic acid (Bio-AA), or the like.

The acid may be, for example, one selected from the group consisting of sulfuric acid, hydrochloric acid, phosphoric acid, carbonic acid and nitric acid.

In the process of recovering 3-hydroxypropionic acid according to one embodiment of the invention, after stirring the acid and the solution containing 3-hydroxypropionate crystal to form a slurry containing the precipitate and 3-hydroxypropionic acid, the precipitate is filtered from the slurry, and the 3-hydroxypropionic acid contained in the filtrate can be recovered. For example, the filtration may be carried out using a filtration flask and a vacuum pump.

Meanwhile, the precipitate may be represented by the following structural formula 3.

[Structural Formula 3]     Cation(Anion)·pH$_2$O

in the structural formula 3, the Cation is the cation of the alkali metal salt or alkali earth metal salt, the Anion is the anion of the acid, and the p is the number of water molecules in a hydrate, which is an integer of 1 or more.

The cation may be, for example, Na$^+$, Mg$^{2+}$ or Ca$^{2+}$, but when it is Mg$^{2+}$ or Ca$^{2+}$, a precipitate can be formed more effectively. Further, the anion may be SO$_4^{2-}$, PO$_4^{3-}$ or CO$_3^{2-}$, but is not limited thereto.

Further, the particle size of the precipitate may be 1.0 $\mu$m or more, 1.5 $\mu$m or more, 10.0 $\mu$m or more, 13.0 $\mu$m or more, or 15.0 $\mu$m or more, and may be 300.0 $\mu$m or less, 250.0 $\mu$m or less, 200.0 $\mu$m or less, 150.0 $\mu$m or less, 100.0 $\mu$m or less, 50.0 $\mu$m or less, 30.0 $\mu$m or less, 20.0 $\mu$m or less, 8.0 $\mu$m or less, 7.0 $\mu$m or less, 6.0 $\mu$m or less, 5.0 $\mu$m or less, or 2.0 $\mu$m or less. The precipitate satisfies the above-mentioned particle size, and thus, the moisture content of the precipitate can be lowered, which can increase the fluidity of a slurry containing the precipitate and facilitate filtration of the precipitates from the slurry. For example, the particle size of the precipitate may be 10.0 $\mu$m or more, and in such a case, the filtration separation from the slurry becomes very easy, and the dust generated when recycling to other industrial fields such as cement production is reduced, which can be very advantageous in processing.

The precipitate may exhibit various particle shapes such as angular, spherical, plate-shape, and needle-shape. Further, the particle size of the precipitate is measured by a scanning electron microscopy (SEM). At this time, the particle size of the precipitate is measured based on the straight-line distance between crystal planes with the longest distance among the straight-line distances between crystal planes contained in the precipitate.

In addition, the moisture content of the precipitate may be 150% or less, 130% or less, 110% or less, 100% or less, 80% or less, or 70% or less, and may be 1% or more, 10% or more, or 30% or more. If the moisture content of the precipitate is too high, the fluidity of the slurry containing the precipitate is lowered, which makes it difficult to proceed with the process, and makes it difficult to separate the precipitate from the slurry, thus making it difficult to recover 3-hydroxypropionic acid in high yield.

The moisture content of the precipitate can be calculated by substituting the weight before drying and the weight after drying of the precipitate into the following Equation 1.

Moisture content (wt.%) = (Weight of precipitate before drying - Weight after drying of precipitate) / (Weight of precipitate after drying) * 100     [Equation 1]

In the process of recovering 3-hydroxypropionic acid according to one embodiment of the invention, the precipitate separated from the slurry may be subjected to a first washing, and the used first washing liquid can be reused.

When the 3-hydroxypropionic acid contained in the filtrate is further recovered after filtering the washing liquid without reusing the washing liquid, the filtered washing liquid is also further contained in the final production solution, which may cause the problem that the concentration of 3-hydroxypropionic acid in the final production solution is very low.

However, in the process of recovering 3-hydroxypropionic acid according to one embodiment of the invention, as the washing liquid is reused during the recovery process, a decrease in the concentration of 3-hydroxypropionic acid can be prevented, and the 3-hydroxypropionic acid contained in the washing liquid is circulated during the process, so that the loss of 3-hydroxypropionic acid can be prevented, and 3-hydroxypropionic acid can be recovered at a high recovery rate.

Further, in the process of recovering 3-hydroxypropionic acid according to one embodiment of the invention, after subjecting the precipitate to a first washing, the first washed precipitate is subjected to a second washing, and the used second washing liquid can be reused.

Meanwhile, the washing water for washing the precipitate is not limited thereto, and may be at least one selected from the group consisting of distilled water, a hydroxyl group-containing solution, or an amine group-containing solution. It is preferable to use distilled water to reduce the formation of by-products. Meanwhile, the distilled water may be distilled water, double distilled water, or triple distilled water. The hydroxy group-containing solution may be alcohol, ethyl alcohol, methyl alcohol or isopropyl alcohol. The amine group-containing solution may be ammonia water, dimethylamine, trimethylamine, or aniline.

The volume ratio of the first washing liquid and the second washing liquid used for washing the precipitate may be 1:1.5 to 1:10.0, 1:2 to 1:7, or 1:3 to 1:5.

The separated precipitate may include 3-hydroxypropionic acid, 3-hydroxypropionate, 3-hydroxypropionate crystal and other impurities. In the first washing of the precipitate, the 3-hydroxypropionic acid and 3-hydroxypropionate having high solubility may be first contained in the first washing liquid and recovered.

Therefore, in order to recover the 3-hydroxypropionic acid and 3-hydroxypropionate at a high concentration, the first washing liquid can be used in a smaller volume than the second washing liquid. That is, as the first washing liquid and the second washing liquid are used in a volume ratio of 1:1.5 to 1:10.0, the first washing liquid containing the 3-hydroxypropionic acid and 3-hydroxypropionate at high concentrations can be recovered through the first washing step.

After that, in the second washing step, the remaining 3-hydroxypropionic acid and 3-hydroxypropionate and other impurities contained in the first washed precipitate can be removed. Moreover, in order to recover the high-purity precipitate, the second washing liquid can be used in a larger volume than the first washing liquid.

The step of reusing the first washing liquid and/or the second washing liquid is not limited thereto, and for example, at least one washing liquid selected from the first washing liquid and the second washing liquid can be reused as a solvent in the step of preparing the solution containing 3-hydroxypropionate crystal.

As mentioned above, the solution containing 3-hydroxypropionate crystal can be prepared by dissolving the 3-hydroxypropionate crystal separated from the concentrate in a solvent such as distilled water, and the first washing liquid and/or the second washing liquid can be reused as a solvent for dissolving the 3-hydroxypropionate crystal.

Further, since the first washing liquid contains 3-hydroxypropionic acid and 3-hydroxypropionate at a high concentration, the first washing liquid can be reused as a solvent for dissolving the 3-hydroxypropionate crystal. Consequently, it is possible to prevent the loss of 3-hydroxypropionic acid generated during the recovery process, and to increase the recovery rate of 3-hydroxypropionic acid.

Further, the process of recovering 3-hydroxypropionic acid will be described in detail below, but before the step of forming 3-hydroxypropionate crystal, the process may further comprise fermenting a bacterium strain having 3-hydroxypropionic acid-producing ability to produce a 3-hydroxypropionic acid fermentation liquor, and concentrating the fermentation liquid to form the concentrate containing 3-hydroxypropionic acid.

Further, at least one washing liquid selected from the first washing liquid and the second washing liquid can be reused in the step of producing the 3-hydroxypropionic acid fermentation liquid.

Since the second washing liquid also contains 3-hydroxypropionic acid and 3-hydroxypropionate, this second washing liquid can be reused in the step of producing the 3-hydroxypropionic acid fermentation liquid.

Since the filtration steps further progress after the step of producing the fermentation liquid, the second washing liquid containing impurities can be reused in the step of producing the fermentation liquid, and the first washing liquid containing 3-hydroxypropionic acid and 3-hydroxypropionate at a high concentration can be reused in the step of preparing the solution containing 3-hydroxypropionate crystal.

Meanwhile, since the first washing liquid and the second washing liquid may contain other impurities in addition to 3-hydroxypropionic acid, the washing liquid is further filtered, and then the filtered washing liquid can be reused during the recovery process.

In addition, at least one washing liquid selected from the first washing liquid and the second washing liquid is mixed with the filtrate in which the precipitate has been filtered to thereby prepare a mixed liquid, and the 3-hydroxypropionic acid can be recovered from the mixed liquid.

When the slurry composition is filtered (solid-liquid separation), it is separated into a precipitate and a filtrate, and 3-hydroxypropionic acid is contained in the filtrate, so that 3-hydroxypropionic acid can be recovered therefrom. Meanwhile, as mentioned above, the first washing liquid and/or the second washing liquid also contain 3-hydroxypropionic acid remaining on the surface of the precipitate, so that the first washing liquid and/or the second washing liquid are mixed with the filtrate. When 3-hydroxypropionic acid is recovered from the mixed liquid, the recovery rate of 3-hydroxypropionic acid can be improved.

The recovery rate of 3-hydroxypropionic acid in the process of recovering 3-hydroxypropionic acid according to one embodiment of the invention may be 40% or more, 50% or more, 60% or more, 70% or more, 80% or more, or 90% or more, for example, 40 to 99.9%, 50 to 99.9%, 60 to 99.9%, 70 to 99.9%, 80 to 99.9%, 90 to 99.9%, 40 to 99%, 50 to 99%, 60 to 99%, 70 to 99%, 80 to 99%, 90 to 99%, 40 to 97%, 50 to 97%, 60 to 97%, 70 to 97%, 80 to 97%, 90 to 97%, 40 to 95%, 50 to 95%, 60 to 95%, 70 to 95%, 80 to 95%, or 90 to 95%, but is not limited thereto. The recovery rate may be calculated based on the weight.

In the process of recovering 3-hydroxypropionic acid according to one embodiment of the invention, the moisture removed through processes such as concentration and evaporation can be reused during the washing process. The moisture removed through processes such as concentration and evaporation was generally discarded as wastewater. However, in the process of recovering 3-hydroxypropionic acid, moisture removed through processes such as concentration and evaporation can be reused during the washing process to thereby reduce the amount of wastewater generated.

For example, the step of concentrating the fermentation liquid to form a concentrate containing the 3-hydroxypropionic acid is carried out by evaporating the fermentation liquid, and moisture removed by the evaporation can be liquefied and reused for at least one washing selected from the first washing and the second washing.

Further, the process of recovering 3-hydroxypropionic acid may further comprise drying the first and second washed precipitate. Therefore, the moisture removed by the drying can be liquefied and reused for at least one washing selected from the first washing and the second washing.

Furthermore, after stirring the acid and the solution containing 3-hydroxypropionate crystal to form a slurry containing a precipitate and 3-hydroxypropionic acid, the filtrate in which the slurry has been filtered can be purified through an ion exchange method, and the like. The purified solution containing 3-hydroxypropionic acid can be concentrated to prepare a concentrated 3-hydroxypropionic acid solution.

At this time, the concentration is carried out through evaporation, and the moisture removed by the evaporation can be liquefied and reused for at least one washing selected from the first washing and the second washing.

Meanwhile, in the process for recovering 3-hydroxypropionic acid according to one embodiment of the invention, 3-hydroxypropionate crystal can be formed from the concentrate containing 3-hydroxypropionic acid in the presence of an alkali metal salt or an alkali earth metal salt, before preparing a solution containing 3-hydroxypropionate crystal separated from the concentrate.

The concentrate containing 3-hydroxypropionic acid may contain 3-hydroxypropionic acid at a concentration of 300 g/L or more, 350 g/L or more, 400 g/L or more, 450 g/L or more, or 500 g/L or more, and may contain 3-hydroxypropionic acid at a concentration of 900 g/L or less, 850 g/L or less, or 800 g/L or less. Whether or not the crystal of 3-hydroxypropionate is formed appears to be affected by the presence of an alkali metal salt or an alkali earth metal salt, the concentration of 3-hydroxypropionic acid in the concentrate, and the like.

Further, when the concentration of the crystal of 3-hydroxypropionic acid in the concentrate is higher than the water solubility of the crystal of 3-hydroxypropionic acid, the crystal of 3-hydroxypropionic acid can be more easily produced. For example, the water solubility of $Ca(3HP)_2$, which is a crystal of 3-hydroxypropionic acid, is 450 g/L at room temperature, and thus, when the concentration of 3-hydroxypropionic acid in the concentrate exceeds 450 g/L, the formation of $Ca(3HP)_2$ crystal can be promoted. Further, the water solubility of $Mg(3HP)_2$, which is a crystal of 3-hydroxypropionic acid, is 250 g/L at room temperature, and thus, when the concentration of 3-hydroxypropionic acid in the concentrate exceeds 250 g/L, the formation of $Mg(3HP)_2$ crystal can be promoted.

The 3-hydroxypropionic salt crystal can be formed from a concentrate satisfying the above-mentioned concentration even while containing the alkali metal salt or the alkali earth metal salt. The alkali metal salt or alkali earth metal salt may be selected without limitation within the purpose range for forming 3-hydroxypropionate crystal. For example, the alkali metal salt or alkali earth metal salt may include one or more cations selected from the group consisting of $Na^+$, $Mg^{2+}$

and $Ca^{2+}$, but when a cation of $Mg^{2+}$ or $Ca^{2+}$ or a salt thereof is used, 3-hydroxypropionic salt crystal can be formed more effectively. For example, the alkali earth metal salt may be $Ca(OH)_2$, $Mg(OH)_2$, or a mixture thereof.

The alkali metal salt or alkali earth metal salt is added and remains in the process of producing the 3-hydroxypropionic acid fermentation liquid, or it can be added in the process of forming 3-hydroxypropionate crystal in the concentrate containing 300 g/L or more of 3-hydroxypropionic acid. Further, the concentration of the alkali metal salt or alkali earth metal salt may be 10% to 100%, or 30% to 90% of the 3-hydroxypropionic acid concentration, and for example, it can be present in the concentrate at a concentration of 10 to 900 g/L, 50 to 800 g/L, 100 to 700 g/L, or 200 to 600 g/L.

Further, the step of forming 3-hydroxypropionate crystal in the concentrate containing 3-hydroxypropionic acid may further comprise contacting the concentrate of 3-hydroxypropionic acid with a nonsolvent. When contacting the concentrate with the nonsolvent, the 3-hydroxypropionate crystal can be more easily formed. When the concentrate of the formed 3-hydroxypropionic acid is brought into contact with a nonsolvent in the presence of the alkali metal salt or the alkali earth metal salt, pure 3-hydroxypropionate crystals can be formed by controlling the crystal formation rate as compared to crystals formed at a high rate due to overconcentration, and thereby, the crystal size can be increased. Such 3-hydroxypropionate crystals contain very low amounts of impurities inside the crystals, and have excellent filterability due to uniform crystal formation, so that not only it is easy to purify, but it is also excellent in shape stability and heat stability in the crystal state, thereby being able to efficiently mass-produce 3-hydroxypropionic acid having high purity.

For example, when a nonsolvent is brought into contact with a concentrate of the formed 3-hydroxypropionic acid in the presence of an alkali metal salt or an alkali earth metal salt, an alkali metal salt or an alkali earth metal salt of 3-hydroxypropionic acid is produced, and as the concentration of the produced alkali metal salt or the alkali earth metal salt of 3-hydroxypropionic acid increases, fine crystals are formed, and solid-liquid phase separation occurs. After that, as crystallization proceeds, the alkali metal salt or the alkali earth metal salt of 3-hydroxypropionic acid grows into solid crystals (3-hydroxypropionate crystal), and the resultant 3-hydroxypropionate crystal contain impurities at a very low content while being separated from liquid impurities such as glycerol and 1,3-propanediol. At this time, the nonsolvent serves to promote crystallization of the alkali metal salt or the alkali earth metal salt of 3-hydroxypropionic acid, so that the solid-liquid separation ability is increased and 3-hydroxypropionate crystal having higher purity can be produced.

The volume ratio between the concentrate of 3-hydroxypropionic acid and the nonsolvent may be determined by considering the concentration of 3-hydroxypropionic acid or the volume of the concentrate and nonsolvent. For example, the concentrate and the nonsolvent may be used in a volume ratio of 1:0.5 to 1:20, or 1:0.5 to 1:10, or 1:0.8 to 1:8, or 1:1 to 1:5.

If the volume of the nonsolvent is too small compared to the concentrate, the concentration at which crystals are formed is high, and the rate of crystal formation is increased, so that pure crystal particles are not formed slowly and irregular crystal particles are rapidly formed. Further, the solid-liquid separation ability is low, which may make it difficult to separate liquid impurities from the alkali metal salt or the alkali earth metal salt of 3-hydroxypropionic acid to be purified. In addition, if the volume of the nonsolvent is too large compared to the concentrate, the formed crystals may be dissolved and dissolved in some cases due to the solubility of the nonsolvent, and the time in the crystal filtration process after crystal formation increases, and the amount of waste liquid increases, which may be uneconomical.

The nonsolvent may include an alcohol-based nonsolvent, a ketone-based nonsolvent, a nitrile-based non-solvent, or a mixture of two or more thereof, and more specifically, at least one alcohol-based nonsolvent may be used.

The ketone-based nonsolvent may include one or more selected from the group consisting of acetone, methyl ethyl ketone, cyclohexanone, diethyl ketone, acetophenone, methyl isobutyl ketone, methylisoamylketone, isophorone and di-(isobutyl)ketone. The alcohol-based nonsolvent may include one or more selected from the group consisting of methanol, ethanol, allyl alcohol, 1-propanol, 2-propanol, 1-butanol, 2-butanol, isobutanol, benzyl alcohol, cyclohexanol, diacetone alcohol, ethylene glycol monoethyl ether, diethylene glycol monomethyl ether, diethylene glycol monoethyl ether, ethylene glycol monobutyl ether, diethylene glycol monobutyl ether, 2-methoxyethanol and 1-decanol. The nitrile-based nonsolvent may include one or more selected from the group consisting of acetonitrile, propionitrile, butyronitrile, valeronitrile, caprylonitrile, heptanenitrile, cyclopentane carbonitrile, cyclohexane carbonitrile, 2-fluorobenzonitrile, 4-fluorobenzonitrile, difluorobenzonitrile, trifluorobenzonitrile, phenylacetonitrile, 2-fluorophenylacetonitrile, and 4-fluorophenylacetonitrile.

The step of contacting the concentrate with a nonsolvent to form 3-hydroxypropionate crystal may be carried out at a temperature of 0°C or more, 15°C or more, 30°C or more, 35°C or more, 40°C or more, 45°C or more, 50°C or more, or 55°C or more, and may be carried out at a temperature of 100°C or less, 90°C or less, 80°C or less, 75°C or less, 70°C or less, 65°C or less, or 0°C to 100°C.

At this time, the temperature may be adjusted to the above range by adding a non-solvent at the above-described temperature to the concentrate, or by heating or cooling in a state in which the concentrate and the nonsolvent are mixed.

The 3-hydroxypropionate crystal may be the form shown in the following structural formula 1 or structural formula 2. That is, the 3-hydroxypropionate crystal may include 3-hydroxypropionate in the form shown in the structural formula 1 or structural formula 2.

In the structural formula 1 and structural formula 2, Cation means a cation, 3HP means 3-hydroxypropionic acid that binds to the cation, n is the number of 3HP that binds to the cation and means an integer of 1 or more, and in the structural formula 2, m is the number of water molecules that binds to Cation(3HP)n in the hydrate, which is an integer of 1 or more. The cation may be, for example, $Na^+$, $Mg^{2+}$ or $Ca^{2+}$, but in the case of $Mg^{2+}$ or $Ca^{2+}$, 3-hydroxypropionate crystal may be formed more effectively.

[Structural Formula 1]        Cation(3HP)$_n$

[Structural Formula 2]        Cation(3HP)$_n$·mH$_2$O

In addition, the step of forming 3-hydroxypropionate crystal may further comprise stirring the concentrate.

The stirring step can be carried out at a temperature of 0 to 70 degrees Celsius, 0 to 60 degrees Celsius, 0 to 50 degrees Celsius, 0 to 40 degrees Celsius, 0 to 35 degrees Celsius, 0 to 30 degrees Celsius, 10 to 70 degrees Celsius, 10 to 60 degrees Celsius, 10 to 50 degrees Celsius, 10 to 40 degrees Celsius, 10 to 35 degrees Celsius, 10 to 30 degrees Celsius, 15 to 70 degrees Celsius, 15 to 60 degrees Celsius, 15 to 50 degrees Celsius, 15 to 40 degrees Celsius, 15 to 35 degrees Celsius, 15 to 30 degrees Celsius, 20 to 70 degrees Celsius, 20 to 60 degrees Celsius, 20 to 50 degrees Celsius, 20 to 40 degrees Celsius, 20 to 35 degrees Celsius, or 20 to 30 degrees Celsius (e.g., room temperature), and/or under conditions of 100 to 2000rpm, 100 to 1500rpm, 100 to 1000rpm, 100 to 500rpm, 100 to 400rpm, or 200 to 400rpm (e.g., about 300rpm).

The particle size distribution $D_{50}$ of the 3-hydroxypropionate crystal may be 20 $\mu$m or more and 90 $\mu$m or less, 25 $\mu$m or more and 85 $\mu$m or less, 30 $\mu$m or more and 80 $\mu$m or less, 35 $\mu$m or more and 75 $\mu$m or less.

In addition, the particle size distribution $D_{10}$ of the 3-hydroxypropionate crystal may be 5 $\mu$m or more and 40 $\mu$m or less, 8 $\mu$m or more and 35 $\mu$m or less, 10 $\mu$m or more and 30 $\mu$m or less, and the particle size distribution $D_{90}$ of the 3-hydroxypropionate crystal may be 50 $\mu$m or more and 200 $\mu$m or less, 60 $\mu$m or more and 190 $\mu$m or less, 65 $\mu$m or more and 180 $\mu$m or less, 70 $\mu$m or more and 175 $\mu$m or less.

The particle size distributions $D_{50}$, $D_{10}$ and $D_{90}$ mean particle sizes at which cumulative volumes of particles reach 50%, 10% and 90%, respectively, in the particle size distribution curve of the particles, wherein the $D_{50}$, $D_{10}$ and $D_{90}$ can be measured using, for example, a laser diffraction method. The laser diffraction method can generally measure particle sizes ranging from a submicron range to several millimeters, and can obtain results with high reproducibility and high resolvability.

If the particle size distributions $D_{50}$, $D_{10}$ and $D_{90}$ of the 3-hydroxypropionate crystal is too large, impurities that must be removed during crystallization may be contained in the crystals, which results in a reduction in purification efficiency. If the particle size distributions are too small, the liquid permeability during filtration of the crystals may be lowered.

Meanwhile, the $(D_{90}\text{-}D_{10})/D_{50}$ of the 3-hydroxypropionate crystal may be 1.00 or more and 3.00 or less, 1.20 or more and 2.80 or less, 1.40 or more and 2.60 or less, or 1.60 or more and 2.40 or less.

Further, the 3-hydroxypropionate crystal may have a volume average particle size of 30 $\mu$m or more and 100 $\mu$m or less, 35 $\mu$m or more and 95 $\mu$m or less, or 40 $\mu$m or more and 90 $\mu$m or less, a number average particle size of 1 $\mu$m or more and 30 $\mu$m or less, 3 $\mu$m or more and 25 $\mu$m or less, or 5 $\mu$m or more and 20 $\mu$m or less, and a volume average particle size of 10 $\mu$m or more and 70 $\mu$m or less, 15 $\mu$m or more and 60 $\mu$m or less, or 20 $\mu$m or more and 55 $\mu$m or less.

If the volume average particle size, number average particle size, and volume average particle size of the 3-hydroxypropionate crystal are too large, impurities that must be removed during crystallization may be contained in the crystals, which results in a reduction in purification efficiency. If they are too small, the liquid permeability during filtration of the crystals may be lowered.

Further, the LW ratio (length to width ratio) and average LW ratio in the particle size distribution ($D_{10}$, $D_{50}$, $D_{90}$) of the 3-hydroxypropionate crystal are 0.50 or more and 3.00 or less, 0.70 or more, 2.80 or less, and 1.00 or more and 2.50 or less. If the LW ratio of the 3-hydroxypropionate crystals is too large, problems of fluidity and clogging may occur during transfer of the crystals, and if the LW ratio is too small, the liquid permeability during filtration of the crystals may be lowered.

The 3-hydroxypropionate crystal can measure the moisture content contained in the crystals by the Karl Fischer method, and the moisture content contained in the 3-hydroxypropionate crystal may be 200 ppm or more and 5000 ppm or less, 250 ppm or more and 4800 ppm or less, 300 ppm or more and 4600 ppm or less, or 350 ppm or more and 4400 ppm or less.

At this time, the moisture contained in the 3-hydroxypropionate crystal means the attached moisture contained between the crystals, rather than the crystal moisture (for example, Ca(3HP)$_2$·2H$_2$O). Further, if the moisture content contained in the 3-hydroxypropionate crystal is too high, it may be recovered in the form of a slurry rather than a crystalline solid, or impurities may be contained in the water, which may cause of a problem of a decrease in purity improvement.

In the process of recovering 3-hydroxypropionic acid according to one embodiment of the invention, as 3-hydroxypropionic acid is prepared through a process such as fermenting a bacterium strain having 3-hydroxypropionic acid-producing ability as described below, the 3-hydroxypropionate crystal may contain a radioactive carbon isotope

($^{14}$C).

The radioactive carbon isotope ($^{14}$C) contains about 1 atom per $10^{12}$ carbon atoms in earth's atmosphere and has a half-life of about 5700 years, and carbon stocks can become abundant in the upper atmosphere due to nuclear reactions in which cosmic rays and normal nitrogen ($^{14}$N) participate. Meanwhile, in fossil fuels, isotopes have broken long ago, so that the $^{14}$C ratio may be substantially zero. When bio-derived raw materials are used as the 3-hydroxypropionic acid raw material, or fossil fuels are used together with it, the content of radioactive carbon isotopes (pMC; percent modern carbon) and the content of bio-carbon contained in 3-hydroxypropionic acid can be measured according to the standard of ASTM D6866-21,

After carbon atoms contained in the compound to be measured is made into graphite form or carbon dioxide gas form, the content can be measured, for example, by the mass spectrometer, or can be measured according to liquid scintillation analysis method. At this time, the two radioactive isotopes can be separated using an accelerator for separating $^{14}$C ions from $^{12}$C ions together with the mass spectrometer, and the content and the content ratio can be measured by a mass spectrometer.

The 3-hydroxypropionate crystal has a radiocarbon isotope content of 20 pMC (percent modern carbon) or more, 50 pMC or more, 90 pMC or more, 100 pMC or more, and a biocarbon content of 20 wt.% or more, 50 wt.% or more, 80 wt.%, 90 wt.%, or 95 wt.%, as measured according to the standard of ASTM D6866-21.

The radiocarbon isotope ratio (pMC) means the ratio of the radiocarbon isotope ($^{14}$C) contained in the 3-hydroxypropionate crystals to the radiocarbon isotope ($^{14}$C) of the current standard reference material, and it can be greater than 100% because the nuclear test program of the 1950s is still in effect and not extinguished.

Further, the content of biocarbon means the content of biocarbon relative to the total carbon content contained in the 3-hydroxypropionate crystal. As this value is larger, it may correspond to an environmentally friendly compound.

Meanwhile, if the radiocarbon isotope content (pMC) and biocarbon content of the 3-hydroxypropionate crystal are too low, the environmental friendliness is reduced, which may not be seen as a bio-derived material.

The crystalline state of the 3-hydroxypropionate crystal can be confirmed through peaks and the like in an X-ray diffraction (XRD) graph.

For example, the 3-hydroxypropionate crystal may exhibit peaks between crystal lattices in the $2\theta$ value range of 8 to 22° during X-ray diffraction (XRD) analysis.

For example, when the concentrate contains magnesium hydroxide ($Mg(OH)_2$), and the formed 3-hydroxypropionate crystal is $Mg(3HP)_2$, peaks between the crystal lattices due to the bond between 3-hydroxypropionic acid and magnesium may appear in the 2θ value range of 8 to 15° during X-ray diffraction (XRD) analysis for $Mg(3HP)_2$. Such peaks show different results from the X-ray diffraction (XRD) analysis results for magnesium hydroxide ($Mg(OH)_2$) or magnesium sulfate ($Mg(SO_4)$). As a result of the X-ray diffraction (XRD) analysis, it can be confirmed that when a specific peak appears in the 2θ value range of 8 to 15°, $Mg(3HP)_2$ crystal is formed.

Specifically, during X-ray diffraction (XRD) analysis for $Mg(3HP)_2$, 3 or more, 4 or more, or 5 or more peaks may appear in the 2θ value range of 8 to 15°, and for example, peaks may appear in the 2θ value range of 8.2 to 9.3°, 9.5 to 11.0°, 11.2 to 12.7°, 12.9 to 13.3°, and 13.5 to 14.8°, respectively.

Further, when calcium hydroxide ($Ca(OH)_2$) is contained in the concentrate and the 3-hydroxypropionate crystal formed therefrom are $Ca(3HP)_2$, peaks between the crystal lattices due to the bond between 3-hydroxypropionic acid and calcium may appear in the 2θ value range of 10 to 22° during X-ray diffraction (XRD) analysis for $Ca(3HP)_2$. Such peaks show different results from the X-ray diffraction (XRD) analysis results for calcium hydroxide ($Ca(OH)_2$) or calcium sulfate ($Ca(SO_4)$). As a result of the X-ray diffraction (XRD) analysis, it can be confirmed that when a specific peak appears in the 2θ value range of 10 to 22°, $Ca(3HP)_2$ crystal is formed.

Specifically, during X-ray diffraction (XRD) analysis for $Ca(3HP)_2$, 3 or more, 5 or more, 7 or more or 9 or more peaks may appear in the 2θ value range of 10 to 22°, and for example, peaks may appear in the 2θ value range of 10.0 to 11.0°, 11.1 to 11.6°, 11.6 to 12.5°, 12.7 to 13.6°, 13.8 to 16.0°, 17.0 to 18.0°, 19.0 to 19.8°, 20.2 to 21.2°, or 21.5 to 22.0°, respectively.

Meanwhile, the incident angle (θ) means the point at which a first differential value (slope of the tangent line, dy/dx) is 0 on a graph where the horizontal axis (x-axis) in the x-y plane is a value of two times the incident angle ($2\theta$) of the incident X-ray, and the vertical axis (y-axis) in the x-y plane is a diffraction intensity, wherein as the value ($2\theta$) of two times the angle of incidence of the incident X-ray, which is the horizontal axis (x-axis), increases in the positive direction, the first differential value (slope of the tangent line, dy/dx) of two times ($2\theta$) the incident angle of X-rays, which is the horizontal axis (x-axis), with respect to the diffraction intensity, which is the vertical axis (y-axis), changes from a positive value to a negative value.

Further, the 3-hydroxypropionate crystal may have a distance (d value) between atoms in the crystals derived from X-ray diffraction (XRD) analysis of 1.00 Å or more and 15.00 Å or less, 1.50 Å or more and 13.00 Å or less, 2.00 Å or more and 11.00 Å or less, 2.50 Å or more and 10.00 Å or less.

For example, when the 3-hydroxypropionate crystal is $Mg(3HP)_2$, a distance (d value) between atoms in the crystals of the peak appearing in the 2θ value range of 8 to 15° may be 1.00 Å or more and 15.00 Å or less, 2.00 Å or more and 13.00 Å or less, 4.00 Å or more and 11.00 Å or less, 5.50 Å or more and 10.00 Å or less.

Further, when the 3-hydroxypropionate crystal is Ca(3HP)$_2$, the distance (d value) between atoms in the crystals of the peak appearing in the 28 value range of 10 to 22° may be 1.00 Å or more and 15.00 Å or less, 2.00 Å or more and 13.00 Å or less, 3.00 Å or more and 10.00 Å or less, 3.40 Å or more and 9.00 Å or less, or 4.00 Å or more and 8.50 Å or less.

Further, the 3-hydroxypropionate crystal may have a glass transition temperature of -55°C or more and -30°C or less, a melting point of 30°C or more and 170°C or less, and a crystallization temperature of 25°C or more and 170°C or less.

The glass transition temperature, melting point, and crystallization temperature may be measured by a differential scanning calorimetry (DSC) for the 3-hydroxypropionate crystal, wherein the heating rate during measurement may be 1 to 20°C/min. Further, the 3-hydroxypropionate crystal ate may have a glass transition temperature of -55°C or more and -30°C or less, -50°C or more and -35°C or less, or - 45°C or more and -40 °C or less. Further, the melting point of the 3-hydroxypropionate crystal may be 30°C or more and 170°C or less, 31°C or more and 160°C or less, 32°C or more and 150°C or less. Further, the crystallization temperature of the 3-hydroxypropionate crystal may be 25°C or more and 170 °C or less, 27°C or more and 160°C or less, or 30°C or more and 150°C or less. In addition, the crystallization stability section of the 3-hydroxypropionate crystal may be -40°C to 150°C.

The purity of 3-hydroxypropionate contained in the 3-hydroxypropionate crystal may be calculated as a percentage (%) of the mass of the compound having the structural formula 1 and/or structural formula 2 relative to the mass of the total crystal recovered. For example, the purity of 3-hydroxypropionate contained in the 3-hydroxypropionate crystal may be 70% or more, 80% or more, 90% or more, 70 to 99.9%, 80 to 99.9%, 90 to 99.9%, 70 to 99%, 80 to 99%, or 90 to 99%, but is not limited thereto.

As mentioned above, the process of recovering 3-hydroxypropionic acid may comprise fermenting a bacterium strain having 3-hydroxypropionic acid-producing ability to produce a 3-hydroxypropionic acid fermentation liquor, and concentrating the fermentation liquid to form a concentrate containing 300 g/L of more of 3-hydroxypropionic acid, prior to the step of forming the 3-hydroxypropionate crystal.

The bacterium strain having 3-hydroxypropionic acid-producing ability may include genes encoding at least one or two proteins selected from the group consisting of glycerol dehydratase and aldehyde dehydrogenase.

In one example, the 3-hydroxypropionic acid-producing strain may further include a gene (gdrAB) encoding glycerol dehydratase reactivator (GdrAB). In one example, the 3-hydroxypropionic acid-producing strain may be a bacterium strain that is further capable of biosynthesizing vitamin B12.

The glycerol dehydratase may be encoded by dhaB (GenBank accession no. U30903.1) gene, but is not limited thereto. The dhaB gene may be an enzyme derived from *Klebsiella pneumonia,* but is not limited thereto. The gene encoding the glycerol dehydratase may include a gene encoding dhaB1, dhaB2 and/or dhaB3. The glycerol dehydratase protein and the gene encoding it may include mutations in the gene and/or amino acid sequence within the range of maintaining an enzyme activity that decomposes glycerol to 3-hydroxypropanal (3-HPA) and water (H$_2$O).

The gene (aldH) encoding the aldehyde dehydrogenase (ALDH) may be, for example, aldH (GenBank Accession no. U00096.3; EaldH) gene derived from *Escherichia coli* or *E. coli* K12 MG1655 cell line, puuC gene derived from *Klebsiella pneumoniae,* and/or KGSADH gene from *Azospirillum brasilense,* but is not limited thereto. The aldehyde dehydrogenase protein and the gene encoding it may include mutations in the gene and/or amino acid sequence within the range of maintaining the activity for producing 3-hydroxypropionic acid from 3-hydroxypropanal.

The medium for producing the fermentation liquid can be selected without limitation within the range for the purposes for producing 3-hydroxypropionic acid. In one example, the medium may contain glycerol as a carbon source. In another example, the medium may be crude glycerol and/or pretreated crude glycerol, but is not limited thereto. In one example, the production medium may further include vitamin B12.

In the step of fermenting a bacterium strain having 3-hydroxypropionic acid-producing ability to produce a 3-hydroxypropionic acid fermentation liquor, the concentration of 3-hydroxypropionic acid contained in the 3-hydroxypropionic acid fermentation liquor may be 1 to 200 g/L, 10 to 150 g/L, 30 to 130 g/L, or 40 to 100 g/L.

Further, the fermentation may be a neutral fermentation, for example, it may be maintained in the pH range of 6 to 8, 6.5 to 8, 6 to 7.5, or 6.5 to 7.5 during fermentation, but is not limited thereto. The pH range can be appropriately adjusted as needed. The alkali metal salt or the alkali earth metal salt may be added for the neutral fermentation. The alkali earth metal salt may include Mg$^{2+}$, Ca$^{2+}$ or a mixture thereof. Further, the alkali earth metal salt may be Ca(OH)$_2$ or Mg(OH)$_2$, but is not limited thereto.

The process of recovering 3-hydroxypropionic acid according to one embodiment of the invention may further include removing (separating) cells from the fermentation liquid; purifying and/or decolorizing the fermentation liquid and/or the fermentation liquid from which cells has been removed; and/or filtering the fermentation liquid and/or the fermentation liquid from which cells have been removed, after the step of producing the 3-hydroxypropionic acid fermentation liquid.

Removal (separation) of the cells may be carried out by selecting methods known in the art without limitation within the range of cell (strain) removal purposes. In one example, the separation of the cells may be carried out by centrifugation.

The step of purifying and/or decolorizing the fermentation liquid and/or the fermentation liquid from which cells have been removed can be carried out by selecting methods known in the art without limitation within the range of the purpose of purification of the fermentation liquid. For example, the step can be carried out by mixing activated carbon with the fermentation liquid and then removing the activated carbon, but is not limited thereto.

The step of filtering the fermentation liquid and/or the fermentation liquid from which cells have been removed can be carried out by selecting methods known in the art without limitation within the range of the purposes of removal of solid impurities, removal of proteins and/or materials with hydrophobic functional groups, and/or decoloration. For example, the step can be carried out by filter filtration and/or activated carbon filtration methods, but is not limited thereto.

The process of recovering 3-hydroxypropionic acid according to one embodiment of the invention may comprise concentrating the fermentation liquid to form the concentrate containing 3-hydroxypropionic acid, after the step of fermenting the bacterium strain having 3-hydroxypropionic acid-producing ability to produce a 3-hydroxypropionic acid fermentation liquid.

Concentration of the fermentation liquid may be carried out by evaporating the fermentation liquid (e.g., a liquid component of the fermentation liquid).

The concentration may be carried out by any means commonly available for evaporating the liquid component of the fermentation liquid. For example, the concentration may be carried out by rotary evaporation, evaporation concentration, vacuum concentration, reduced pressure concentration, and the like, but is not limited thereto.

In one example, the concentration of 3-hydroxypropionic acid in the fermentation liquid after concentration may be increased by 2 to 50 times, 2 to 40 times, 2 to 30 times, 2 to 20 times, 2 to 10 times, 5 to 50 times, 5 to 40 times, 5 to 30 times, 5 to 20 times, or 5 to 10 times compared to before concentration.

According to another embodiment of the present invention, provided is a 3-hydroxypropionic acid-containing slurry composition comprising a precipitate represented by the following structural formula 4 and 3-hydroxypropionic acid.

[Structural Formula 4]  $Cation(Anion) \cdot pH_2O$

In the structural formula 4, the Cation is the cation of the alkali metal salt or alkali earth metal salt, and for example, it may be $Na^+$, $Mg^{2+}$ or $Ca^{2+}$, but when it is $Mg^{2+}$ or $Ca^{2+}$, 3-hydroxypropionate crystal can be formed more effectively.

Further, the Anion is an anion of the acid, and may be, for example, $SO_4^{2-}$, $PO_4^{3-}$ or $CO_3^{2-}$, but is not limited thereto. The p is the number of water molecules in a hydrate, which is an integer of 1 or more.

The slurry composition may be formed in the process of recovering 3-hydroxypropionic acid according to one embodiment of the invention, and for example, it may be formed in the step of adding an acid to 3-hydroxypropionate crystal separated from the concentrate at a temperature of, for example, 30°C or more and 90°C or less.

The particle size of the precipitate may be 1.0 $\mu$m or more, 1.5 $\mu$m or more, 10.0 $\mu$m or more, 13.0 $\mu$m or more, or 15.0 $\mu$m or more, and may be 300.0 $\mu$m or less, 250.0 $\mu$m or less, 200.0 $\mu$m or less, 150.0 $\mu$m or less, 100.0 $\mu$m or less, 50.0 $\mu$m or less, 30.0 $\mu$m or less, 20.0 $\mu$m or less, 8.0 $\mu$m or less, 7.0 $\mu$m or less, 6.0 $\mu$m or less, or 5.0 $\mu$m or less, 2.0 $\mu$m or less. The precipitate satisfies the above-mentioned particle size, and thus, the moisture content of the precipitate can be lowered, which can increase the fluidity of a slurry containing the precipitate and facilitate filtration of the precipitates from the slurry. For example, the particle size of the precipitate may be 10.0 $\mu$m or more, and in this case, filtration separation from the slurry becomes very easy, and dust generated when recycling to other industrial fields such as cement production is reduced, which can be very advantageous in processing.

The precipitate may exhibit various particle shapes such as angular, spherical, plate-shape, and needle-shape. Further, the particle size of the precipitate can be measured by a scanning electron microscopy (SEM). At this time, the particle size of the precipitate may be measured based on the straight-line distance between crystal planes with the longest distance among the straight-line distances between crystal planes contained in the precipitate.

In addition, the moisture content of the precipitate may be 150% or less, 130% or less, 110% or less, 100% or less, 80% or less, 70% or less, and 1% or more, 10% or more, 30% or more. If the moisture content of the precipitate is too high, the fluidity of the slurry containing the precipitate is lowered, which makes it difficult to proceed with the process, and makes it difficult to separate the precipitate from the slurry, thus making it difficult to recover 3-hydroxypropionic acid in high yield.

## ADVANTAGEOUS EFFECTS

The process of recovering 3-hydroxypropionic acid provided by the present invention can easily separate fermentation by-products and/or additives through crystallization of 3-hydroxypropionate, thereby recovering high-purity 3-hydroxypropionate. The recovered 3-hydroxypropionate crystal is reacted with an acid, and the precipitate is separated from the prepared slurry to thereby recover 3-hydroxypropionic acid in high purity and high yield, and at the same time, the washing water used for washing the precipitate can be reused in the process to thereby recover 3-hydroxypropionic acid in high concentration, high purity and high yield.

## BRIEF DESCRIPTION OF THE DRAWINGS

FIG. 1 is a scanning electron microscope (SEM) photograph of the precipitate formed in Example 3.
FIG. 2 is a scanning electron microscope (SEM) photograph of the precipitate formed in Comparative Example 3.
FIG. 3 is a scanning electron microscope (SEM) photograph of the precipitate formed in Example 4.
FIG. 4 is a scanning electron microscope (SEM) photograph of the precipitate formed in Example 5.

## DETAILED DESCRIPTION OF THE EMBODIMENTS

Hereinafter, the present invention will be described in more detail with reference to examples. However, the following examples are for illustrative purposes only, and are not intended to limit the scope of the present invention.

**Preparation Example 1: Preparation of bacterium strain for producing 3-hydroxypropionic acid**

Recombinant vectors into which genes encoding glycerol dehydratase and aldehyde dehydrogenase, which are known to produce 3-hydroxypropionic acid (3HP) using glycerol as a substrate, were introduced were manufactured. The prepared recombinant vector was introduced into *E.coli* W3110 strain to prepare a 3-hydroxypropionic acid-producing strain.

More specifically, a BtuR gene encoding adenosyltransferase was cloned into plasmid pCDF containing a gene (dhaB) encoding glycerol dehydratase, a gene (aldH) encoding aldehyde dehydrogenase and a gene (gdrAB) encoding glycerol dehydratase reactivase. The resulting pCDF_J23101_dhaB_gdrAB_J23100_aldH_btuR vector was introduced into strain W3110 (KCCM 40219) by an electroporation method using an electroporation device (Bio-Rad, Gene Pulser Xcell) to prepare 3-hydroxypropionic acid-producing strain. The process of preparing the 3-hydroxypropionic acid-producing strain of Preparation Example 1 and the vectors, primers, and enzymes used were carried out with reference to Example 1 of Korean Unexamined Patent Publication No. 10-2020-0051375,

**Preparation Example 2: Preparation of Ca(3HP)$_2$ Crystal**

The 3-hydroxypropionic acid-producing strain prepared in Preparation Example 1 was fermented and cultured at 35°C in a 5L fermenter using unpurified glycerol as a carbon source to produce 3-hydroxypropionic acid. In order to prevent the lowering of pH due to the production of 3-hydroxypropionic acid, calcium hydroxide (Ca(OH)$_2$), which is an alkali earth metal salt, was added to maintain the pH to be neutral during the fermentation.

After fermentation culture, cells were removed by centrifugation (4000 rpm, 10 minutes, 4°C), and primary fermentation liquid purification (primary purification) was performed using activated carbon. Specifically, activated carbon was added to the fermentation liquid from which bacterial cells were removed by centrifugation, the mixture was well mixed, and then centrifuged again to separate the activated carbon. Then, the fermentation liquid from which the activated carbon was separated was filtered with a vacuum pump through a 0.7 $\mu$m filter paper to purify the 3-hydroxypropionic acid fermentation liquid.

The concentration of 3-hydroxypropionic acid in the fermentation liquid after completion of the primary purification was a level of 50 to 100 g/L, and the fermentation liquid was concentrated to a concentration of 600 g/L using a rotary evaporator (50°C, 50 mbar) to prepare a concentrate, and ethanol was added in an amount of two times the volume of the concentrate, and stirred (300 rpm) at room temperature to produce Ca(3HP)$_2$ crystals. At this time, the concentration of the alkali earth metal salt in the concentrate was 493.3 g/L (based on Ca(OH)$_2$). The resulting crystals were washed three times with ethanol (EtOH) and dried in an oven at 50°C to finally recover the crystals.

**Comparative Example 1**

5.0 g of Ca(3HP)$_2$ crystal recovered in Preparation Example 2 was added to 13.5 ml of distilled water to prepare a Ca(3HP)$_2$ aqueous solution, which was stirred at a temperature of 60°C and 350 rpm for 10 minutes. 2.5 g of a 95% sulfuric acid solution was added to the Ca(3HP)$_2$ aqueous solution at a uniform rate for 5 minutes so that the molar ratio of sulfur (S) to calcium (Ca) is 0.9, and further stirred for 30 minutes to form a slurry containing CaSO$_4$ precipitate and 3-hydroxypropionic acid.

Filtration was performed using a filtration flask and a vacuum pump in order to separate the CaSO$_4$ precipitate. Then, a filtrate (B) before washing was obtained in a filtering flask, and then the filtered CaSO$_4$ precipitate was washed with 20 ml of distilled water, and then filtered to obtain a filtrate (C) after washing. Additionally, the result was washed with 40 ml of distilled water, and then filtered to obtain a second washing liquid (D). Then, the CaSO$_4$ precipitate was dried in an oven

**EP 4 324 817 B1**

at a temperature of 40°C for 20 hours to finally obtain the dried $CaSO_4$ precipitate.

**Example 1**

A filtrate before washing (G), a first washing liquid (H), a second wash liquid (I) and a dried $CaSO_4$ precipitate were obtained in the same manner as in Comparative Example 1, except that in the preparation of a $Ca(3HP)_2$ aqueous solution, the first washing liquid (C) of Comparative Example 1 was reused instead of 13.5 ml of distilled water.

**<Test Example>**

**1. Measurement of the concentration of 3-hydroxypropionic acid**

For the filtrate before washing (B, G), first washing liquid (C, H), and second washing liquid (D, I) obtained in Example 1 and Comparative Example 1, the concentration of 3-hydroxypropionic acid was measured using a high-performance liquid chromatography (HPLC), and the results are shown in Table 1 below.

**2. Measurement of recovery rate of 3-hydroxypropionic acid**

The recovery rate of 3-hydroxypropionic acid obtained in Example 1 and Comparative Example 1 was measured and calculated using a high-performance liquid chromatography (HPLC), and the results are shown in Table 1 below.

Specifically, the absolute amount ($Y_{ref}$) of 3-hydroxypropionic acid in the $Ca(3HP)_2$ aqueous solution, the content ($Y_{filtration}$) of 3-hydroxypropionic acid contained in the filtrate (B or G) before washing, the content ($Y_{washing1}$) of 3-hydroxypropionic acid contained in the first washing liquid (C or H) and the content ($Y_{washing2}$) of 3-hydroxypropionic acid contained in the second washing liquid (D or I) were measured by a high performance liquid chromatography (HPLC), which were substituted into the following Equations 2 to 4, respectively, to calculate the recovery rate of 3-hydroxypropionic acid.

Recovery rate (%) of 3-hydroxypropionic acid in the filtrate (B or G) before washing = $Y_{filtration}/Y_{ref}$ * 100     [Equation 2]

Recovery rate (%) of 3-hydroxypropionic acid in the first washing liquid (C or H) = $Y_{washing1}/Y_{ref}$ * 100     [Equation 3]

Recovery rate (%) of 3-hydroxypropionic acid in the second washing liquid (D or I) = $Y_{washing2}/Y_{ref}$ * 100     [Equation 4]

In addition, assuming that 3-hydroxypropionic acid that was not recovered with the filtrate before washing and the first and second washing liquids remained in the $CaSO_4$ precipitate, this was shown in Table 1 below as the recovery rate of 3-hydroxypropionic acid contained in the $CaSO_4$ precipitate.

[Table 1]

| | | Concentration of 3-hydroxypropionic acid (g/L) | Recovery rate of 3-hydroxypropionic acid (%) |
|---|---|---|---|
| Comparative Example 1 | Filtrate before washing (B) | 338.4 | 61.9 |
| | First washing liquid (C) | 63.0 | 23.0 |
| | Second washing liquid (D) | 8.5 | 6.4 |
| | $CaSO_4$ precipitate | - | 8.7 |
| Example 1 | Filtrate before washing (G) | 382.2 | 66.0 |
| | First washing liquid (H) | 73.9 | 24.8 |
| | Second washing liquid (I) | 10.4 | 7.1 |
| | $CaSO_4$ precipitate | - | 2.1 |

Referring to Table 1, it was confirmed that since Example 1 reused the first washing liquid (C) of Comparative

**15**

Example 1 for preparing a Ca $(3HP)_2$ aqueous solution, Example 1 and Comparative Example 1 used the same amount of Ca $(3HP)_2$ crystals, but Example 1 showed significantly higher 3-hydroxypropionic acid concentrations in all filtrates and washing liquids compared to Comparative Example 1. This can be expected to be because 3-hydroxypropionic acid is not lost during the process but is circulated within the process.

**Example 2**

6.12 g of Ca $(3HP)_2$ crystal recovered in Preparation Example 2 was added to 13 ml of distilled water to prepare 32.0 wt.% Ca $(3HP)_2$ aqueous solution, which was stirred at a temperature of 60°C and 350 rpm for 10 minutes.

2.5 g of a 95% sulfuric acid solution was added to the Ca $(3HP)_2$ aqueous solution at a uniform rate for 5 minutes so that the molar ratio of sulfur (S) to calcium (Ca) is 0.85, and further stirred for 30 minutes to form a slurry containing $CaSO_4$ precipitate and 3-hydroxypropionic acid.

Filtration was performed using a filtration flask and a vacuum pump in order to separate the $CaSO_4$ precipitate. Then, a filtrate (A) before washing was obtained in a filtering flask, and then the filtered $CaSO_4$ precipitate was washed with 30 ml of distilled water, and then filtered to obtain a filtrate (B) after washing. Then, the $CaSO_4$ precipitate was dried in an oven at a temperature of 40°C for 20 hours to finally obtain the dried $CaSO_4$ precipitate. Additionally, filtrates (A) and (B) containing 3-hydroxypropionic acid were obtained.

**Example 3**

A process of recovering 3-hydroxypropionic acid was carried out in the same manner as in Example 2, except that the filtered $CaSO_4$ precipitate was washed with 250 ml of distilled water, instead of washing the filtered $CaSO_4$ precipitate with 30 ml of distilled water.

**Comparative Example 2**

A process of recovering 3-hydroxypropionic acid was carried out in the same manner as in Example 2, except that a Ca $(3HP)_2$ aqueous solution was stirred at a temperature of 25°C, instead of stirring the Ca $(3HP)_2$ aqueous solution at a temperature of 60°C.

**Comparative Example 3**

A process of recovering 3-hydroxypropionic acid was carried out in the same manner as in Example 3, except that a Ca $(3HP)_2$ aqueous solution was stirred at a temperature of 25°C, instead of stirring the Ca $(3HP)_2$ aqueous solution at a temperature of 60°C.

**<Test Example>**

**1. Evaluation of removal rate of calcium (Ca) element**

When converting the 3-hydroxypropionate crystal (Ca $(3HP)_2$ crystal) in Example 3 and Comparative Example 3 to 3-hydroxypropionic acid, $CaSO_4$ precipitates were produced, and thus, the content of the calcium(Ca) element removed was measured by an inductively coupled plasma analysis.

Specifically, the content ($X_{ref}$) of calcium(Ca) element contained in the 32.0 wt. % Ca $(3HP)_2$ aqueous solution, and the content ($X_{filtration}$) of calcium(Ca) element contained in the filtrate (B) after washing were respectively analyzed, and the removal rate of the calcium (Ca) element was calculated according to the following Equation 5, and the results are shown in Table 2 below.

[Equation 5]

$$\text{Removal rate of calcium(Ca) element (\%)} = (X_{ref} - X_{filtration})/X_{ref} * 100$$

**2. Measurement of particle size of precipitate**

The particle size of the $CaSO_4$ precipitate formed in Example 3 and Comparative Example 3 was confirmed through a scanning electron microscope analysis, and the results are shown in Table 2 below.

At this time, the particle size of the $CaSO_4$ precipitate was measured based on the straight-line distance between crystal planes with the longest distance among the straight-line distances between crystal planes contained in the precipitate.

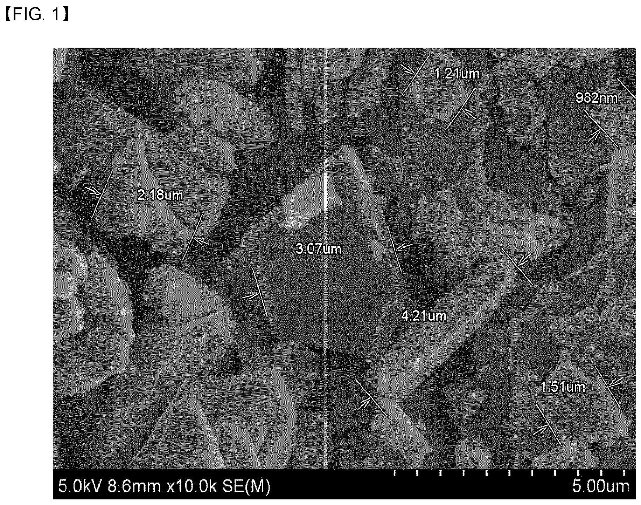

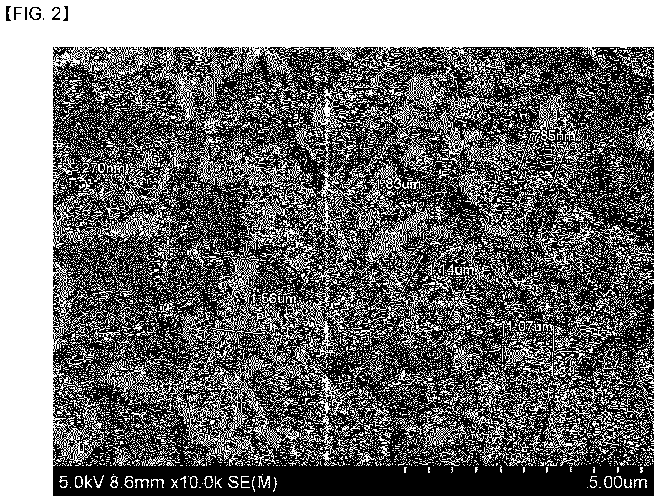

On the other hand, FIG. 1 is a scanning electron microscope (SEM) photograph of the precipitate formed in Example 3, and FIG. 2 is a scanning electron microscope (SEM) photograph of the precipitate formed in Comparative Example 3.

### 3. Measurement of moisture content of precipitate

The weight before drying and the weight after drying of the $CaSO_4$ precipitate formed in Example 3 and Comparative Example 3 were substituted into the following Equation 6 to calculate the moisture content of the precipitate, and the results are shown in Table 2 below.

$$\text{Moisture content (wt.\%)} = (\text{Weight of precipitate before drying - Weight of precipitate after drying}) / (\text{Weight of precipitate after drying}) * 100 \qquad \text{[Equation 6]}$$

### 4. Measurement of recovery rate of 3-hydroxypropionic acid

The recovery rate of 3-hydroxypropionic acid obtained in Example 2 and Comparative Example 2 was measured by a high-performance liquid chromatography (HPLC), and the results are shown in Table 3 below.

Specifically, the content ($Y_{ref}$) of 3-hydroxypropionic acid contained in the 32.0 wt.% $Ca(3HP)_2$ aqueous solution, the content ($Y_{filtration1}$) of 3-hydroxypropionic acid in the filtrate (A) before washing, and the content ($Y_{filtration2}$) of 3-hydroxypropionic acid in the filtrate (B) after washing were measured by a high performance liquid chromatography (HPLC), which were respectively substituted into the following Equations 7 and 8 to calculate the recovery rates of 3-hydroxypropionic acid in the filtrate (A) before washing and the filtrate (B) after washing.

$$\text{Recovery rate (\%) of 3-hydroxypropionic acid in the filtrate (A) before washing (\%)} = Y_{filtration1}/Y_{ref} * 100 \qquad \text{[Equation 7]}$$

$$\text{Recovery rate (\%) of 3-hydroxypropionic acid in the filtrate (B) after washing} = Y_{filtration2}/Y_{ref} * 100 \qquad \text{[Equation 8]}$$

[Table 2]

| | Removal rate of calcium (Ca) element (%) | Particle size of precipitate ($\mu$m) | Moisture content of precipitate (wt.%) |
|---|---|---|---|
| Example 3 | 73.1 | 1.5-4.5 | 68.0 |
| Comparative Example 3 | 76.8 | 0.2-2.0 | 175.0 |

[Table 3]

| | | Recovery rate of 3-hydroxypropionic acid (%) |
|---|---|---|
| Example 2 | Filtrate (A) before washing | 62.5 |
| | Filtrate (B) after washing | 28.4 |
| | Subtotal | 90.9 |
| Comparative Example 2 | Filtrate (A) before washing | 34.7 |
| | Filtrate (B) after washing | 32.5 |
| | Subtotal | 67.2 |

Referring to Tables 2 and 3, it was confirmed that the $CaSO_4$ precipitate formed in Example 3 in which sulfuric acid was charged at a temperature of 60°C had a larger particle size and a significantly lower moisture content than the $CaSO_4$ precipitate formed in Comparative Example 3 in which sulfuric acid was charged at a temperature of 25°C. In addition, it was confirmed that Example 2, in which sulfuric acid was charged at a temperature of 60°C, had a significantly higher recovery rate of 3-hydroxypropionic acid than Comparative Example 2, in which sulfuric acid was charged at a temperature of 25°C.

**Example 4**

5.8 g of Ca(3HP)$_2$ crystal recovered in Preparation Example 2 wase added to 9.2 ml of distilled water, and stirred for 1 hour to prepare a Ca(3HP)$_2$ aqueous solution (concentration of 630 g/L).

10.9 ml of distilled water was charged into a new flask, and heated up to a temperature of 60°C using a heating mantle. After that, 240 mg of sulfuric acid was divided into 24 mg each and charged 10 times at intervals of 6 minutes into a flask containing the distilled water, and at the same time, the Ca(3HP)$_2$ aqueous solution was charged into the flask containing the distilled water at a rate of 0.25 g/min for 1 hour, and then stirred. Then, the result was further stirred for 30 minutes to form a slurry containing CaSO$_4$ precipitate and 3-hydroxypropionic acid.

After that, the CaSO$_4$ precipitate and the filtrate were separated using a filtration flask and a vacuum pump.

**Example 5**

5.8 g of Ca(3HP)$_2$ crystal recovered in Preparation Example 2 were added to 9.2 ml of distilled water, and stirred for 1 hour to prepare a Ca(3HP)$_2$ aqueous solution.

25.7 ml of distilled water was added to a new flask, and heated up to a temperature of 60°C using a heating mantle. Then, 240 mg of sulfuric acid was divided into 48 mg each and charged 5 times at intervals of 12 minutes into the flask containing the distilled water, and at the same time, the Ca(3HP)$_2$ aqueous solution was charged into the flask containing the distilled water at a rate of 0.25 g/min for 1 hour, and then stirred. Then, the result was further stirred for 30 minutes to form a slurry containing CaSO$_4$ precipitate and 3-hydroxypropionic acid.

After that, the CaSO$_4$ precipitate and the filtrate were separated using a filtration flask and a vacuum pump.

**<Test Example>**

**1. Measurement of particle size of precipitate**

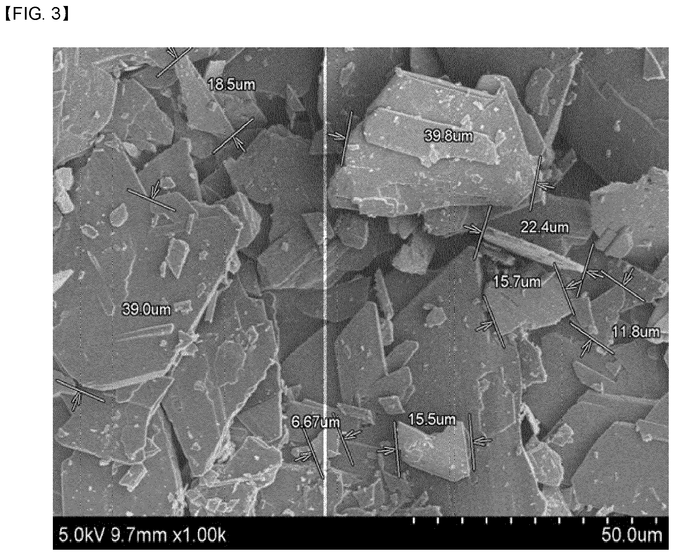

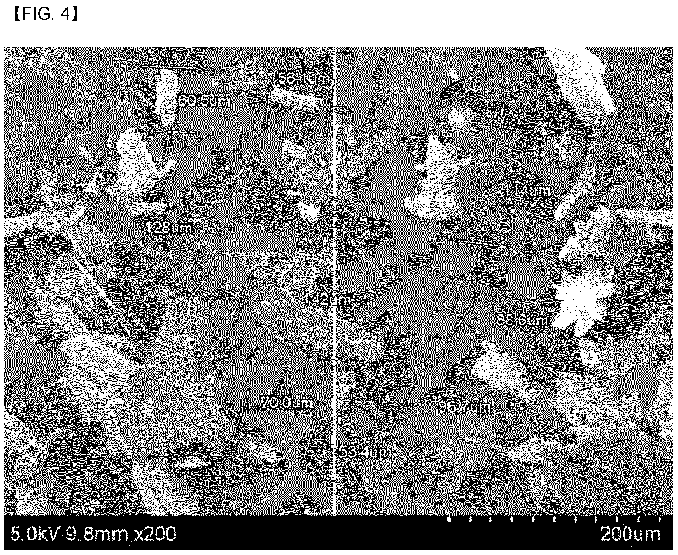

The particle size of the CaSO$_4$ precipitate recovered in Examples 4 and 5 was confirmed through a scanning electron microscope analysis, and the results are shown in Table 4 below. At this time, the particle size of the CaSO$_4$ precipitate was measured based on the straight-line distance between crystal planes with the longest distance among the straight-line distances between crystal planes contained in the precipitate. On the other hand, FIG. 3 is a scanning electron microscope (SEM) photograph of the precipitate formed in Example 4, and FIG. 4 is a scanning electron microscope (SEM) photograph of the precipitate formed in Example 5.

**2. Measurement of recovery rate of 3-hydroxypropionic acid**

The recovery rate of 3-hydroxypropionic acid in Examples 4 and 5 was measured by a high performance liquid chromatography (HPLC), and the results are shown in Table 4 below.

Specifically, before being charged into the flask, the content (X) of 3-hydroxypropionic acid contained in the Ca(3HP)$_2$ aqueous solution, and the content (Y) of 3-hydroxypropionic acid in the filtrate were analyzed by HPLC, and then substituted into the following Equation 9 to calculate the recovery rate of 3-hydroxypropionic acid.

[Equation 9]

Recovery rate (%) of 3-hydroxypropionic acid = Y/X * 100

**3. Evaluation of fluidity of slurry**

The fluidity of the slurries formed in Examples 4 and 5 was evaluated according to the following criteria, and the results are shown in Table 4 below.

<Judgment Criteria>

High: the volume of the slurry remaining inside the flask is 10% or less when pouring and taking out the slurry inside the flask without a washing solvent

Low: the volume of the slurry remaining inside the flask is greater than 10% when pouring and taking out the slurry inside the flask without a washing solvent

## 4. Evaluation of filtration speed of slurry

The filtration speed of the slurries formed in Examples 4 and 5 was evaluated according to the following criteria, and the results are shown in Table 4 below.

<Judgment Criteria>

Fast: the injection flow rate of the filtrate obtained in the filtration flask is 50 ml/min or more

Slow: the injection flow rate of the filtrate obtained in the filtration flask is less than 50 ml/min

[Table 4]

|  | Particle size of precipitate ($\mu$m) | Recovery rate of 3-hydroxypropionic acid (%) | Fluidity | Filtration speed |
|---|---|---|---|---|
| Example 4 | 10~40 | 74.5 | High | Fast |
| Example 5 | 30~140 | 84.1 | High | Fast |

Referring to Table 4, it was confirmed that in the case of the $CaSO_4$ precipitate formed in Examples 4 and 5, in which $Ca(3HP)_2$ aqueous solution was continuously charged and sulfuric acid was divisionally charged, the particle size was large, the fluidity of the precipitate was high, the filtration speed of the precipitate was fast, and the recovery rate of 3-hydroxypropionic acid was as high as 74.5% or more.

## Claims

1. A process of recovering 3-hydroxypropionic acid, comprising:

   forming a 3-hydroxypropionate crystal in a concentrate containing 3-hydroxypropionic acid in the presence of an alkali metal salt or an alkali earth metal salt;
   preparing a solution containing 3-hydroxypropionate crystal separated from the concentrate;
   stirring an acid and the solution containing 3-hydroxypropionate crystal to form a precipitate; and
   subjecting the precipitate to a first washing.

2. The process of recovering 3-hydroxypropionic acid according to claim 1, further comprising reusing a first washing liquid used for the first washing of the precipitate.

3. The process of recovering 3-hydroxypropionic acid according to claim 1, wherein:

   before the stirring an acid and the solution containing 3-hydroxypropionate crystal to form a precipitate,
   the process further comprises adding an acid to the solution containing 3-hydroxypropionate crystal at a temperature of 30°C or more and 90°C or less.

4. The process of recovering 3-hydroxypropionic acid according to claim 1, wherein:

   before the stirring an acid and the solution containing 3-hydroxypropionate crystal to form a precipitate,
   the process further comprises charging the acid and the solution containing 3-hydroxypropionate crystal into a reactor, either continuously or in a state divided in 2 times or more.

5. The process of recovering 3-hydroxypropionic acid according to claim 4, wherein:
   the solution containing 3-hydroxypropionate crystal is continuously charged into the reactor.

6. The process of recovering 3-hydroxypropionic acid according to claim 4, wherein:
the acid is charged into the reactor in a state divided in 3 times or more and 20 times or less.

7. The process of recovering 3-hydroxypropionic acid according to claim 1, wherein:

after the subjecting the precipitate to a first washing,
the process further comprises subjecting the first washed precipitate to a second washing and reusing the used second washing liquid.

8. The process of recovering 3-hydroxypropionic acid according to claim 7, wherein:
at least one washing liquid selected from the first washing liquid and the second washing liquid is reused as a solvent in the preparing of the solution containing 3-hydroxypropionate crystal separated from the concentrate.

9. The process of recovering 3-hydroxypropionic acid according to claim 7, wherein:
at least one washing liquid selected from the first washing liquid and the second washing liquid is mixed with a filtrate in which the precipitate has been filtered to prepare a mixed liquid, and the 3-hydroxypropionic acid is recovered from the mixed liquid.

10. The process of recovering 3-hydroxypropionic acid according to claim 7, further comprising:

fermenting a bacterium strain having 3-hydroxypropionic acid-producing ability to produce a 3-hydroxypropionic acid fermentation liquid; and
concentrating the fermentation liquid to form the concentrate containing 3-hydroxypropionic acid,
wherein at least one washing liquid selected from the first washing liquid and the second washing liquid is reused in the fermenting of a bacterium strain having 3-hydroxypropionic acid-producing ability to produce the 3-hydroxypropionic acid fermentation liquor.

11. The process of recovering 3-hydroxypropionic acid according to claim 10, wherein:

the concentrating of the fermentation liquid to form the concentrate containing 3-hydroxypropionic acid is carried out by evaporating the fermentation liquid, and
the moisture removed by the evaporation is liquefied and reused for at least one washing selected from the first washing and the second washing.

12. The process of recovering 3-hydroxypropionic acid according to claim 7,

further comprising drying the washed precipitate,
wherein the moisture removed by the drying is liquefied and reused for at least one washing selected from the first washing and the second washing.

13. The process of recovering 3-hydroxypropionic acid according to claim 1, wherein:

a particle size of the precipitate is 1.0 $\mu$m or more, and
a moisture content of the precipitate is 150% or less.

14. The process of recovering 3-hydroxypropionic acid according to claim 1, wherein:
the alkali earth metal salt is $Ca(OH)_2$, $Mg(OH)_2$, or a mixture thereof.

15. A slurry composition comprising a precipitate represented by the following structural formula 4 and 3-hydroxypropionic acid.

[Structural Formula 4]        Cation(Anion)·pH$_2$O

wherein,

the Cation is $Na^+$, $Mg^{2+}$ or $Ca^{2+}$,
the Anion is $SO_4^{2-}$, $PO_4^{3-}$ or $CO_3^{2-}$, and
the p is the number of water molecules in a hydrate, which is an integer of 1 or more.

**Patentansprüche**

1.  Verfahren zur Gewinnung von 3-Hydroxypropionsäure, umfassend:

    Bilden eines 3-Hydroxypropionatkristalls in einem Konzentrat, das 3-Hydroxypropionsäure enthält, in Gegenwart eines Alkalimetallsalzes oder eines Erdalkalimetallsalzes;
    Herstellen einer Lösung, die 3-Hydroxypropionatkristall enthält, der von dem Konzentrat getrennt ist;
    Rühren einer Säure und der Lösung, die 3-Hydroxypropionatkristall enthält, um einen Niederschlag zu bilden; und
    Unterziehen des Niederschlags einem ersten Waschen.

2.  Verfahren zur Gewinnung von 3-Hydroxypropionsäure nach Anspruch 1,
    ferner umfassend das Wiederverwenden einer ersten Waschflüssigkeit, die für das erste Waschen des Niederschlags verwendet wird.

3.  Verfahren zur Gewinnung von 3-Hydroxypropionsäure nach Anspruch 1, wobei:

    vor dem Rühren einer Säure und der Lösung, die 3-Hydroxypropionatkristall enthält, um einen Niederschlag zu bilden,
    das Verfahren ferner das Zugeben einer Säure zu der Lösung, die 3-Hydroxypropionatkristall enthält, bei einer Temperatur von 30 °C oder mehr und 90 °C oder weniger umfasst.

4.  Verfahren zur Gewinnung von 3-Hydroxypropionsäure nach Anspruch 1, wobei:

    vor dem Rühren einer Säure und der Lösung, die 3-Hydroxypropionatkristall enthält, um einen Niederschlag zu bilden,
    das Verfahren ferner das Laden der Säure und der Lösung, die 3-Hydroxypropionatkristall enthält, in einen Reaktor entweder kontinuierlich oder in einem 2-fach oder mehr geteilten Zustand umfasst.

5.  Verfahren zur Gewinnung von 3-Hydroxypropionsäure nach Anspruch 4, wobei:
    die Lösung, die 3-Hydroxypropionatkristall enthält, kontinuierlich in den Reaktor geladen wird.

6.  Verfahren zur Gewinnung von 3-Hydroxypropionsäure nach Anspruch 4, wobei:
    die Säure in den Reaktor in einem 3-fach oder mehr und 20-fach oder weniger geteilten Zustand geladen wird.

7.  Verfahren zur Gewinnung von 3-Hydroxypropionsäure nach Anspruch 1, wobei:

    nach dem Unterziehen des Niederschlags einem ersten Waschen,
    das Verfahren ferner das Unterziehen des ersten gewaschenen Niederschlags einem zweiten Waschen und das Wiederverwenden der verwendeten zweiten Waschflüssigkeit umfasst.

8.  Verfahren zur Gewinnung von 3-Hydroxypropionsäure nach Anspruch 7, wobei:
    mindestens eine Waschflüssigkeit, die aus der ersten Waschflüssigkeit und der zweiten Waschflüssigkeit ausgewählt ist, bei der Herstellung der Lösung, die 3-Hydroxypropionatkristall enthält, der von dem Konzentrat getrennt ist, als Lösungsmittel wiederverwendet wird.

9.  Verfahren zur Gewinnung von 3-Hydroxypropionsäure nach Anspruch 7, wobei:
    mindestens eine Waschflüssigkeit, die aus der ersten Waschflüssigkeit und der zweiten Waschflüssigkeit ausgewählt ist, mit einem Filtrat gemischt wird, in dem der Niederschlag gefiltert wurde, um eine gemischte Flüssigkeit herzustellen, und die 3-Hydroxypropionsäure aus der gemischten Flüssigkeit gewonnen wird.

10. Verfahren zur Gewinnung von 3-Hydroxypropionsäure nach Anspruch 7, ferner umfassend:

    Fermentieren eines Bakterienstamms mit der Fähigkeit zur Produktion von 3-Hydroxypropionsäure, um eine 3-Hydroxypropionsäure-Fermentationsflüssigkeit zu produzieren; und
    Konzentrieren der Fermentationsflüssigkeit, um das Konzentrat zu bilden, das 3-Hydroxypropionsäure enthält, wobei mindestens eine Waschflüssigkeit, die aus der ersten Waschflüssigkeit und der zweiten Waschflüssigkeit ausgewählt ist, bei der Fermentierung eines Bakterienstamms mit der Fähigkeit zur Produktion von 3-Hydroxypropionsäure, um die 3-Hydroxypropionsäure-Fermentationsflüssigkeit zu produzieren, wiederverwendet wird.

11. Verfahren zur Gewinnung von 3-Hydroxypropionsäure nach Anspruch 10, wobei:

das Konzentrieren der Fermentationsflüssigkeit, um das Konzentrat zu bilden, das 3-Hydroxypropionsäure enthält, durch Verdampfen der Fermentationsflüssigkeit durchgeführt wird, und
die durch die Verdampfung entfernte Feuchtigkeit verflüssigt und für mindestens ein Waschen, das aus dem ersten Waschen und dem zweiten Waschen ausgewählt ist, wiederverwendet wird.

12. Verfahren zur Gewinnung von 3-Hydroxypropionsäure nach Anspruch 7,

ferner umfassend das Trocknen des gewaschenen Niederschlags,
wobei die durch das Trocknen entfernte Feuchtigkeit verflüssigt und für mindestens ein Waschen, das aus dem ersten Waschen und dem zweiten Waschen ausgewählt ist, wiederverwendet wird.

13. Verfahren zur Gewinnung von 3-Hydroxypropionsäure nach Anspruch 1, wobei:

eine Teilchengröße des Niederschlags 1,0 oder mehr beträgt, und
ein Feuchtigkeitsgehalt des Niederschlags 150 % oder weniger beträgt.

14. Verfahren zur Gewinnung von 3-Hydroxypropionsäure nach Anspruch 1, wobei:
das Erdalkalimetallsalz $Ca(OH)_2$ , $Mg(OH)_2$ oder eine Mischung davon ist.

15. Aufschlämmungszusammensetzung, umfassend einen Niederschlag, der durch die folgende Strukturformel 4 dargestellt ist, und 3-Hydroxypropionsäure.

[Strukturformel 4]    Kation(Anion)·$pH_2O$

wobei

das Kation $Na^+$, $Mg^{2+}$ oder $Ca^{2+}$ ist,
das Anion $SO_4^{2-}$, $PO_4^{3-}$ oder $CO_3^{2-}$ ist und
das p die Anzahl von Wassermolekülen in einem Hydrat ist, die eine ganze Zahl von 1 oder mehr ist.

**Revendications**

1. Procédé de récupération d'acide 3-hydroxypropionique, consistant à :

former un cristal de 3-hydroxypropionate dans un concentré contenant de l'acide 3-hydroxypropionique en présence d'un sel de métal alcalin ou d'un sel de métal alcalino-terreux ;
préparer une solution contenant le cristal de 3-hydroxypropionate séparé du concentré ;
agiter un acide et la solution contenant le cristal de 3-hydroxypropionate pour former un précipité ; et
soumettre le précipité à un premier lavage.

2. Procédé de récupération d'acide 3-hydroxypropionique selon la revendication 1,
consistant en outre à réutiliser un premier liquide de lavage utilisé pour le premier lavage du précipité.

3. Procédé de récupération d'acide 3-hydroxypropionique selon la revendication 1, dans lequel :

avant d'agiter un acide et la solution contenant le cristal de 3-hydroxypropionate pour former un précipité,
le procédé consiste en outre à ajouter un acide à la solution contenant le cristal de 3-hydroxypropionate à une température égale ou supérieure à 30°C et égale ou inférieure à 90°C.

4. Procédé de récupération d'acide 3-hydroxypropionique selon la revendication 1, dans lequel :

avant d'agiter un acide et la solution contenant le cristal de 3-hydroxypropionate pour former un précipité,
le procédé consiste en outre à charger l'acide et la solution contenant le cristal de 3-hydroxypropionate dans un réacteur, soit en continu, soit dans un état divisé en 2 fois ou plus.

5. Procédé de récupération d'acide 3-hydroxypropionique selon la revendication 4, dans lequel :
la solution contenant le cristal de 3-hydroxypropionate est chargée en continu dans le réacteur.

6. Procédé de récupération d'acide 3-hydroxypropionique selon la revendication 4, dans lequel :
l'acide est chargé dans le réacteur dans un état divisé en 3 fois ou plus et 20 fois ou moins.

7. Procédé de récupération d'acide 3-hydroxypropionique selon la revendication 1, dans lequel :

après avoir soumis le précipité à un premier lavage,
le procédé consiste en outre à soumettre le premier précipité lavé à un deuxième lavage et à réutiliser le deuxième liquide de lavage utilisé.

8. Procédé de récupération d'acide 3-hydroxypropionique selon la revendication 7, dans lequel :
au moins un liquide de lavage sélectionné parmi le premier liquide de lavage et le deuxième liquide de lavage est réutilisé comme solvant dans la préparation de la solution contenant le cristal de 3-hydroxypropionate séparé du concentré.

9. Procédé de récupération d'acide 3-hydroxypropionique selon la revendication 7, dans lequel :
au moins un liquide de lavage sélectionné parmi le premier liquide de lavage et le deuxième liquide de lavage est mélangé avec un filtrat dans lequel le précipité a été filtré pour préparer un liquide mélangé, et l'acide 3-hydroxypropionique est récupéré du liquide mélangé.

10. Procédé de récupération d'acide 3-hydroxypropionique selon la revendication 7, consistant en outre à :

faire fermenter une souche bactérienne ayant la capacité de produire de l'acide 3-hydroxypropionique pour produire un liquide de fermentation d'acide 3-hydroxypropionique ; et
concentrer le liquide de fermentation pour former le concentré contenant l'acide 3-hydroxypropionique,
dans lequel au moins un liquide de lavage sélectionné parmi le premier liquide de lavage et le deuxième liquide de lavage est réutilisé dans la fermentation d'une souche bactérienne ayant la capacité de produire de l'acide 3-hydroxypropionique pour produire une liqueur de fermentation d'acide 3-hydroxypropionique.

11. Procédé de récupération d'acide 3-hydroxypropionique selon la revendication 10, dans lequel :

la concentration du liquide de fermentation pour former le concentré contenant l'acide 3-hydroxypropionique est effectuée en évaporant le liquide de fermentation, et
l'humidité extraite par l'évaporation est liquéfiée et réutilisée pour au moins un lavage sélectionné parmi le premier lavage et le deuxième lavage.

12. Procédé de récupération d'acide 3-hydroxypropionique selon la revendication 7,

consistant en outre à sécher le précipité lavé,
dans lequel l'humidité extraite par le séchage est liquéfiée et réutilisée pour au moins un lavage sélectionné parmi le premier lavage et le deuxième lavage.

13. Procédé de récupération d'acide 3-hydroxypropionique selon la revendication 1, dans lequel :

une taille de particule du précipité est égale ou supérieure à 1,0 $\mu$m, et
une teneur en humidité du précipité est égale ou inférieure à 150 %.

14. Procédé de récupération d'acide 3-hydroxypropionique selon la revendication 1, dans lequel :
le sel de métal alcalino-terreux est $Ca(OH)_2$, $Mg(OH)_2$, ou un mélange de ces derniers.

15. Composition de suspension comprenant un précipité représenté par la formule structurelle 4 ci-après et l'acide 3-hydroxypropionique.

[Formule structurelle 4]      Cation(Anion)$\cdot$p$H_2$O

dans lequel,

le Cation est Na$^+$, Mg$^{2+}$ ou Ca$^{2+}$,
l'Anion est SO$_4$$^{2-}$, PO$_4$$^{3-}$ ou CO$_3$$^{2-}$, et
le p est le nombre de molécules d'eau dans un hydrate, lequel est un nombre entier de 1 ou plus.

【FIG. 1】

【FIG. 2】

【FIG. 3】

【FIG. 4】

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020200051375

**Non-patent literature cited in the description**

- *Chemical Engineering Science*, vol. 77, 18-25